# EUROPEAN PATENT APPLICATION

(11) **EP 2 535 409 A1**
(43) Date of publication of application: **19.12.2012**
(21) Application number: 11742284.0
(22) Date of filing: 10.02.2011
(51) Int. Cl.: C12N 15/09, C12N 15/117

(54) **METHOD FOR PRODUCING RECOMBINANT VIRUS**

(30) Priority: 12.02.2010 US 705317
(71) Applicant: Otsuka Pharmaceutical Co., Ltd., Chiyoda-ku Tokyo 101-8535 (JP)
(72) Inventor: KAWASAKI, Masanori, Osaka-shi Osaka 541-0045 (JP); INAGAKI, Katsuya, Osaka-shi Osaka 541-0045 (JP); MIZUKOSHI, Masami, Osaka-shi Osaka 541-0045 (JP)
(74) Representative: Blodig, Wolfgang
(86) International application number: PCT/JP2011/052812
(87) International publication number: WO 2011/099541

(57) **Abstract**

To provide a method for producing, at high purity, a recombinant baculovirus which exhibits intended immunogenicity and is useful for a pharmaceutical (e.g., a vaccine), a transfer vector for use in the production of the recombinant baculovirus, and a method for producing the transfer vector. The method for producing a transfer vector which includes therein a fusion gene containing at least one gene encoding a virus-particle-forming protein and a gene encoding a foreign immunogenic protein, the method including modifying the polynucleotide sequence of the gene encoding the virus-particle-forming protein so as not to alter the amino acid residues constituting a corresponding naturally occurring virus-particle-forming protein and/or modifying the polynucleotide sequence of the gene so that a portion of the amino acid residues constituting the naturally occurring virus-particle-forming protein is deleted.

## Description

### [Technical Field]

The present invention relates to a method for producing a recombinant virus having an intended purity; to a transfer vector for producing the recombinant virus; and to a method for producing the transfer vector. More particularly, the present invention relates to a method for producing a recombinant virus, in which generation of an unintended recombinant is suppressed upon production of a recombinant baculovirus; to a transfer vector for producing the recombinant virus; and to a method for producing the transfer vector.

### [Background Art]

There have been known virus particle genes which can present a foreign immunogenic protein in the form of fusion antigen on virus particles, including baculovirus gp64 gene, vesicular stomatitis virus glycoprotein gene, human immunodeficiency virus type 1 glycoprotein gene, human respiratory syncytial virus membrane glycoprotein gene, influenza A virus hemagglutinin gene, influenza B virus hemagglutinin gene, herpes simplex virus glycoprotein gene, and murine hepatitis virus S protein gene.

Also, there have been known foreign immunogenic protein antigens for vaccines against infections, cancers, etc., including malaria antigen, influenza virus antigen, *Mycobacterium tuberculosis* antigen, SARS virus antigen, West Nile fever virus antigen, dengue fever virus antigen, HIV antigen, HCV antigen, leishmania antigen, trypanosome antigen, leucocytozoon antigen, and cancer antigens such as CEA, WT1, and MAGE. There have also been known immunogenic proteins formed by fusion between a foreign immunogenic protein gene and a gene for a cytokine (e.g., IL-2, IL-12, IL-6, or IFN-γ) present in the human body. A fusion antigen obtained through expression of a fusion between such a virus particle gene and an immunogenic gene has been used for vaccines.

Meanwhile, protein expression systems employing baculovirus have been used for industrial production of proteins of interest. In recent years, baculovirus has been shown to transfer a foreign gene into insect cells and also into mammals, and has been found to serve as a potential vector for gene therapy.

For example, Patent Document 1 discloses a recombinant baculovirus expression vector containing a plurality of independent promoters including a DNA region including, in a promoter derived from a baculovirus early gene, a gene encoding a virus nonstructural protein, and a DNA region including, in a promoter derived from a baculovirus late gene, a gene encoding a virus structural protein.

Patent Document 2 discloses a method for producing a protein by means of a genetic recombination technique employing a baculovirus, in which the baculovirus gp64 gene is bound to a gene encoding a protein of interest; the resultant fusion gene is expressed to thereby produce the protein in the form of being fused with virus particles; the virus particles with which the protein is fused are recovered; and the protein is separated from the virus particles through cleavage.

Patent Document 3 discloses a method for improved transgene expression, the method including optimizing the nucleotide codon usage of exogenous DNA to alter codon usage to that of a host cell type in which the exogenous DNA sequence is to be expressed, so as to express a protein of interest in the host cell.

Patent Document 4 discloses synthetic polynucleotides encoding HPV31L1, wherein the polynucleotides are free from internal transcription termination signals that are recognized by yeast, and have been codon-optimized for high level expression in a yeast cell, which polynucleotides are provided for producing, by use of yeast, protein products expressed by HPV31L1 genes, which have been associated with cervical cancer.

Patent Document 5 discloses a method and device for optimizing a nucleotide sequence for the purpose of expressing a protein, and a codon optimization method suitable for a host cell system for expression.

Patent Document 6 discloses a recombinant *Plutella xylostella* baculovirus for use as an insecticidal agent, which has incorporated within its genome a gene encoding an insecticidal toxin and a signal sequence of a nonhomologous peptide; and an expression cassette encoding an insecticidal toxin wherein a codon-optimized gene encoding the insecticidal toxin has been substituted with an alternative codon that is more effectively utilized by an insect cell protein.

Patent Document 7 discloses a recombinant nucleic acid molecule encoding a fusion protein which is expressed in a listeria bacterium, the nucleic acid molecule including a first polynucleotide encoding a signal sequence of a peptide native to the bacterium, in which the first polynucleotide is codon-optimized for expression in the bacterium, and a second polynucleotide encoding a polypeptide of a foreign antigen, in which the second polynucleotide is in the same translation reading frame as the first polynucleotide, wherein the recombinant nucleic acid molecule encodes a fusion protein containing the signal sequence of the peptide and the polypeptide; an expression cassette; a bacterium containing an expression vector; and a vaccine composition containing the bacterium. This patent document also discloses a recombinant nucleic acid molecule wherein the second polynucleotide encoding a polypeptide of a foreign antigen is codon-optimized for expression in the bacterium.
Thus, genetic codon substitution has been performed for the purpose of increasing or reducing the expression efficiency of a protein of interest in a host.

The present inventors have previously prepared a transfer vector by fusing a polynucleotide encoding the amino acid sequence of an immunogenic protein with a polynucleotide encoding the protein of virus particles of an expression cell, and have reported that a recombinant virus prepared by homologous recombination with the DNA of a virus of interest (e.g., baculovirus) is used in a pharmaceutical composition (e.g., a vaccine) (Patent Document 8).

### [Related Art Document]

### [Patent Document]

[Patent Document 1] Japanese Patent No. 3366328
[Patent Document 2] JP-A-2002-253263
[Patent Document 3] WO 2006/024867 pamphlet
[Patent Document 4] WO 2004/084831 pamphlet
[Patent Document 5] WO 2004/059556 pamphlet
[Patent Document 6] WO 1999/58705 pamphlet
[Patent Document 7] WO 2005/071088 pamphlet
[Patent Document 8] WO 2007/091624 pamphlet

### [Summary of the Invention]

### [Problems to be Solved by the Invention]

An object of the present invention is to provide a method for producing, at high purity, a recombinant virus (in particular, a recombinant baculovirus) which exhibits intended immunogenicity and is useful for a pharmaceutical (e.g., a vaccine), by suppressing generation of an unintended recombinant virus upon production of the recombinant baculovirus. Another object of the present invention is to provide a method for producing a transfer vector employed for production of the recombinant virus.

### [Means for Solving the Problems]

As described above, the present inventors previously applied a patent on a transfer vector which can express, in both insect cells and vertebrate cells, a fusion protein of a foreign immunogenic protein of interest and a protein forming virus particles (hereinafter may be referred to as "virus-particle-forming protein"), as well as a recombinant baculovirus produced by use of the transfer vector (Patent Document 8). However, when a recombinant baculovirus is produced by use of the transfer vector, the thus-produced recombinant baculovirus may contain therein an unintended recombinant baculovirus in which a DNA region of a gene encoding the foreign immunogenic protein of interest has been deleted (hereinafter the unintended recombinant baculovirus may be referred to as "mutant virus"), which may cause a problem in that the purity of a pharmaceutical containing a recombinant baculovirus of interest is reduced.

In order to solve such a problem, the present inventors have conducted extensive studies, and as a result have found that when the polynucleotide sequence of a gene encoding a virus-particle-forming protein, the gene contained in a fusion gene which is included in a transfer vector, is modified so as not to alter the amino acid residues constituting a corresponding naturally occurring virus-particle-forming protein and/or the polynucleotide sequence of the gene is modified so that a portion of the amino acid residues constituting the naturally occurring virus-particle-forming protein is deleted, followed by co-transfection of the transfer vector and baculovirus DNA into insect cells, generation of the aforementioned mutant virus can be considerably suppressed, and thus a recombinant baculovirus of interest can be produced at high purity.

Accordingly, the present invention provides the following 1) to 21).
1) A method for producing a transfer vector which includes therein a fusion gene containing at least one gene encoding a virus-particle-forming protein and a gene encoding a foreign immunogenic protein, the method comprising modifying the polynucleotide sequence of the gene encoding the virus-particle-forming protein so as not to alter the amino acid residues constituting a corresponding naturally occurring virus-particle-forming protein and/or modifying the polynucleotide sequence of the gene so that a portion of the amino acid residues constituting the naturally occurring virus-particle-forming protein is deleted.
2) A method for producing a transfer vector which includes therein a fusion gene containing at least one gene encoding a virus-particle-forming protein and a gene encoding a foreign immunogenic protein, the method comprising modifying the polynucleotide sequence of the gene encoding the virus-particle-forming protein so as not to alter the amino acid residues constituting a corresponding naturally occurring virus-particle-forming protein.
3) A method according to 1) above, which comprises the following steps (a) to (c) and/or (d) and (e):
   (a) a step of modifying the polynucleotide sequence of the gene encoding the virus-particle-forming protein so as not to alter the amino acid residues constituting a corresponding naturally occurring virus-particle-forming protein, and obtaining the nucleic acid sequence information of a polynucleotide including the gene encoding the virus-particle-forming protein;
   (b) a step of synthesizing the polynucleotide on the basis of the nucleic acid sequence information obtained in step (a);
   (c) a step of ligating or inserting, into the polynucleotide synthesized in step (b), a polynucleotide of the gene encoding the foreign immunogenic protein;
   (d) a step of synthesizing a polynucleotide encoding a partial polypeptide of the naturally occurring virus-particle-forming protein obtained through deletion of a portion of the amino acid residues constituting the naturally occurring virus-particle-forming protein, or a polynucleotide whose sequence has been modified so as not to alter the amino acid residues constituting the partial polypeptide; and
   (e) a step of ligating or inserting, into the polynucleotide synthesized in step (d), a polynucleotide of the gene encoding the foreign immunogenic protein.
4) A method according to 1) or 2) above, wherein the fusion gene includes, upstream thereof, a polynucleotide encoding a signal sequence derived from the virus-particle-forming protein.
5) A method according to 1) or 2) above, wherein the gene encoding the virus-particle-forming protein is any of a baculovirus gp64 gene, a vesicular stomatitis virus glycoprotein gene, a human immunodeficiency virus type 1 glycoprotein gene, a human respiratory syncytial virus membrane glycoprotein gene, an influenza A virus hemagglutinin gene, an influenza B virus hemagglutinin gene, a herpes simplex virus glycoprotein gene, and a murine hepatitis virus S protein gene.
6) A method according to 1) or 2) above, wherein the gene encoding the virus-particle-forming protein is a baculovirus gp64 gene.
7) A method according to 1) or 2) above, wherein the gene encoding the foreign immunogenic protein is a gene for an antigen selected from among a malaria antigen, an influenza virus antigen, a *Mycobacterium tuberculosis* antigen, an SARS virus antigen, a West Nile fever virus antigen, a dengue fever virus antigen, an HIV antigen, an HCV antigen, a leishmania antigen, a trypanosome antigen, a leucocytozoon antigen, and a cancer antigen; or a gene for a fusion antigen of a cytokine and at least one gene selected from the group of these antigen genes.
8) A method according to 1) or 2) above, wherein, in the transfer vector, the fusion gene is ligated to the downstream of a dual promoter prepared through ligation between a promoter capable of functioning in vertebrate cells and a baculovirus promoter.
9) A method according to 8) above, wherein the promoter capable of functioning in vertebrate cells is selected from among a cytomegalovirus promoter, an SV40 promoter, a retrovirus promoter, a metallothionein promoter, a heat-shock protein promoter, a CAG promoter, an elongation factor 1α promoter, an actin promoter, a ubiquitin promoter, an albumin promoter, and an MHC class promoter; and the baculovirus promoter is selected from among a polyhedrin promoter, a p10 promoter, an IE1 promoter, a p35 promoter, a p39 promoter, and a gp64 promoter.
10) A method for producing a transfer vector containing a DNA region of a gene encoding a foreign immunogenic protein, the DNA region being inserted between virus DNA regions including at least one gene encoding a virus-particle-forming protein, the method comprising modifying a polynucleotide sequence of an N-terminal and/or C-terminal virus DNA region fused with the DNA region of the gene encoding the foreign immunogenic protein so that the polynucleotide sequence exhibits reduced identity to a naturally occurring polynucleotide sequence of the same virus DNA region; and/or modifying the polynucleotide sequence so that a portion of the amino acid residues constituting a corresponding naturally occurring virus-particle-forming protein is deleted.
11) A method for producing a recombinant baculovirus comprising employing a transfer vector which includes therein a fusion gene containing at least one gene encoding a virus-particle-forming protein and a gene encoding a foreign immunogenic protein, the method comprising co-transfecting, into an insect host cell, baculovirus DNA and the transfer vector produced by modifying the polynucleotide sequence of the gene encoding the virus-particle-forming protein so as not to alter the amino acid residues constituting a corresponding naturally occurring virus-particle-forming protein, and/or modifying the polynucleotide sequence of the gene so that a portion of the amino acid residues constituting the naturally occurring virus-particle-forming protein is deleted.
12) A transfer vector which includes therein a fusion gene containing at least one gene encoding a virus-particle-forming protein and a gene encoding a foreign immunogenic protein, wherein the polynucleotide sequence of the gene encoding the virus-particle-forming protein is modified so as not to alter the amino acid residues constituting a corresponding naturally occurring virus-particle-forming protein and/or the polynucleotide sequence of the gene is modified so that a portion of the amino acid residues constituting the naturally occurring virus-particle-forming protein is deleted.
13) A transfer vector which includes therein a fusion gene containing at least one gene encoding a virus-particle-forming protein and a gene encoding a foreign immunogenic protein, wherein the polynucleotide sequence of the gene encoding the virus-particle-forming protein is modified so as not to alter the amino acid residues constituting a corresponding naturally occurring virus-particle-forming protein.
14) A transfer vector according to 12) above, which is produced by performing the following steps (a) to (c) and/or (d) and (e):
   (a) a step of modifying the polynucleotide sequence of the gene encoding the virus-particle-forming protein so as not to alter the amino acid residues constituting a corresponding naturally occurring virus-particle-forming protein, and obtaining the nucleic acid sequence information of a polynucleotide including the gene encoding the virus-particle-forming protein;
   (b) a step of synthesizing the polynucleotide on the basis of the nucleic acid sequence information obtained in step (a);
   (c) a step of ligating or inserting, into the polynucleotide synthesized in step (b), a polynucleotide of the gene encoding the foreign immunogenic protein;
   (d) a step of synthesizing a polynucleotide encoding a partial polypeptide of the naturally occurring virus-particle-forming protein obtained through deletion of a portion of the amino acid residues constituting the naturally occurring virus-particle-forming protein, or a polynucleotide whose sequence has been modified so as not to alter the amino acid residues constituting the partial polypeptide; and
   (e) a step of ligating or inserting, into the polynucleotide synthesized in step (d), a polynucleotide of the gene encoding the foreign immunogenic protein.
15) A transfer vector according to 12) or 13) above, wherein the fusion gene includes, upstream thereof, a polynucleotide encoding a signal sequence derived from the virus-particle-forming protein.
16) A transfer vector according to 12) or 13) above, wherein the gene encoding the virus-particle-forming protein is any of a baculovirus gp64 gene, a vesicular stomatitis virus glycoprotein gene, a human immunodeficiency virus type 1 glycoprotein gene, a human respiratory syncytial virus membrane glycoprotein gene, an influenza A virus hemagglutinin gene, an influenza B virus hemagglutinin gene, a herpes simplex virus glycoprotein gene, and a murine hepatitis virus S protein gene.
17) A transfer vector according to 12) or 13) above, wherein the gene encoding the virus-particle-forming protein is a baculovirus gp64 gene.
18) A transfer vector according to 12) or 13) above, wherein the gene encoding the foreign immunogenic protein is a gene for an antigen selected from among a malaria antigen, an influenza virus antigen, a *Mycobacterium tuberculosis* antigen, an SARS virus antigen, a West Nile fever virus antigen, a dengue fever virus antigen, an HIV antigen, an HCV antigen, a leishmania antigen, a trypanosome antigen, a leucocytozoon antigen, and a cancer antigen; or a gene for a fusion antigen of a cytokine and at least one gene selected from the group of these antigen genes.
19) A transfer vector according to 12) or 13) above, wherein the fusion gene is ligated to the downstream of a dual promoter prepared through ligation between a promoter capable of functioning in vertebrate cells and a baculovirus promoter.
20) A transfer vector according to 19) above, wherein the promoter capable of functioning in vertebrate cells is selected from among a cytomegalovirus promoter, an SV40 promoter, a retrovirus promoter, a metallothionein promoter, a heat-shock protein promoter, a CAG promoter, an elongation factor 1α promoter, an actin promoter, a ubiquitin promoter, an albumin promoter, and an MHC class promoter; and the baculovirus promoter is selected from among a polyhedrin promoter, a p10 promoter, an IE1 promoter, a p35 promoter, a p39 promoter, and a gp64 promoter.
21) A transfer vector containing a DNA region of a gene encoding a foreign immunogenic protein, the DNA region being inserted between virus DNA regions including at least one gene encoding a virus-particle-forming protein, wherein a polynucleotide sequence of an N-terminal and/or C-terminal virus DNA region fused with the DNA region of the gene encoding the foreign immunogenic protein is modified so that the polynucleotide sequence exhibits reduced identity to a naturally occurring polynucleotide sequence of the same virus DNA region; and/or the polynucleotide sequence is modified so that a portion of the amino acid residues constituting a corresponding naturally occurring virus-particle-forming protein is deleted.

### [Effects of the Invention]

When the transfer vector of the present invention is employed, generation of mutant virus DNA (which is generated through deletion of a DNA region of a gene encoding a foreign immunogenic protein) can be suppressed upon co-transfection for the production of a recombinant virus of interest by a homologous recombinant technique, and the recombinant virus of interest can be produced from a target cell at high purity. Thus, the present invention realizes effective production of a recombinant virus (in particular, a recombinant baculovirus) which is useful for a therapeutic and/or prophylactic drug for, for example, infections (e.g., malaria, influenza, tuberculosis, and hepatitis), cancer, and autoimmune diseases, or useful for cell therapy or vaccine products.

### [Brief Description of the Drawings]

[Fig. 1]

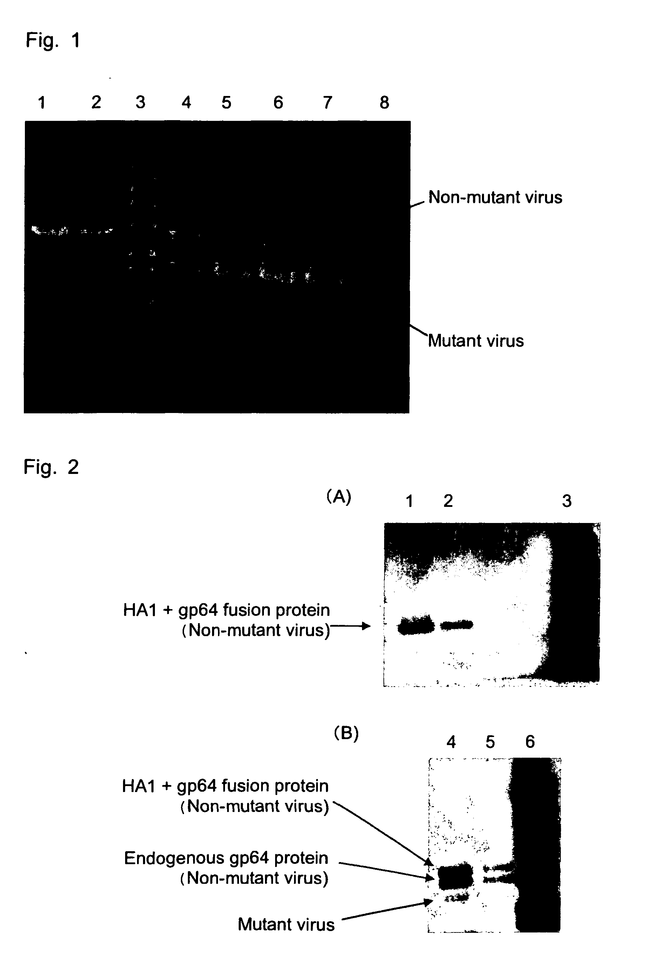

   Fig. 1 shows a decrease in purity of a baculovirus and an increase in amount of mutant virus DNA through subculture.
[Fig. 2]
   Fig. 2 shows that the mutant virus present in a virus solution is a gp64 gene mutant virus.
[Fig. 3]

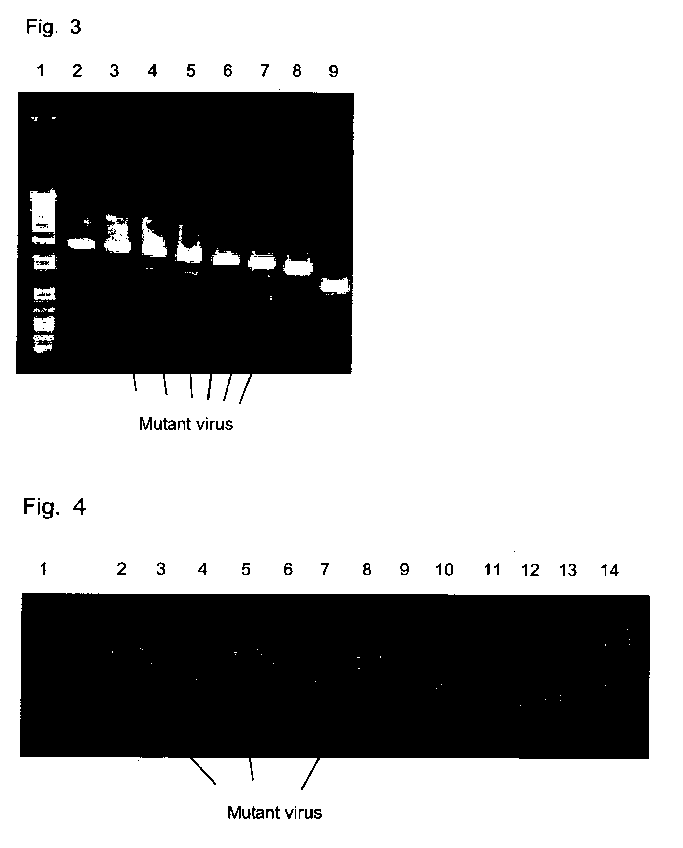

   Fig. 3 shows that a mutant virus is not detected or is difficult to detect in a fusion construct in which a virus DNA region encoding a virus-particle-forming protein has been modified so as not to alter the amino acid residues constituting the protein, or in a fusion construct including a DNA region obtained through deletion of a portion of the aforementioned virus DNA region.
[Fig. 4]
   Fig. 4 shows that, regardless of the type of an antigen gene, a mutant virus is difficult to detect in a fusion construct including a DNA region obtained through deletion of a portion of a virus DNA region encoding a virus-particle-forming protein.
[Fig. 5]

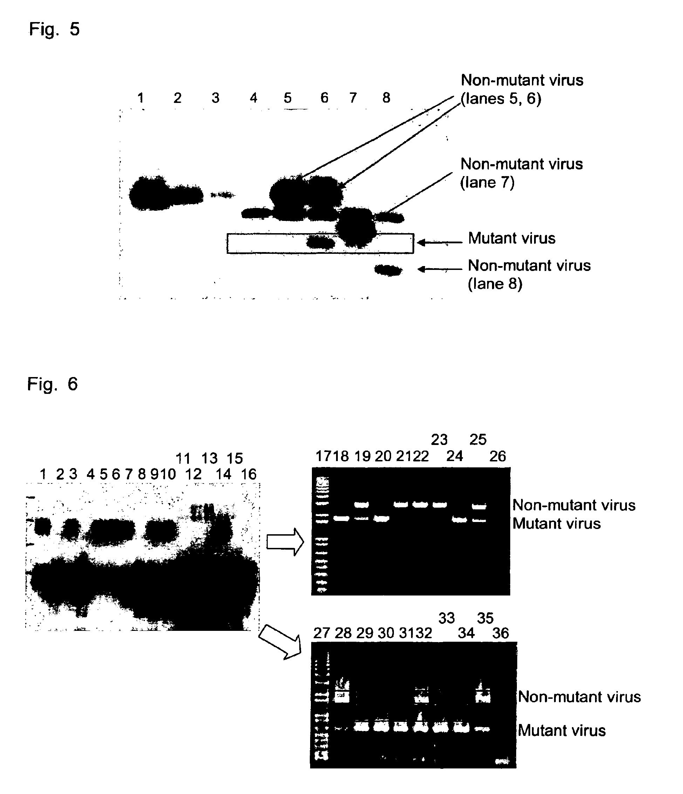

   Fig. 5 shows that a recombinant baculovirus of high purity is produced in a fusion construct in which a virus DNA region encoding a virus-particle-forming protein has been modified so as not to alter the amino acid residues constituting the protein, or in a fusion construct including a DNA region obtained through deletion of a portion of the aforementioned virus DNA region.
[Fig. 6]
   Fig. 6 shows that a mutant virus is generated whether a single promoter or a dual promoter is employed.
[Fig. 7]

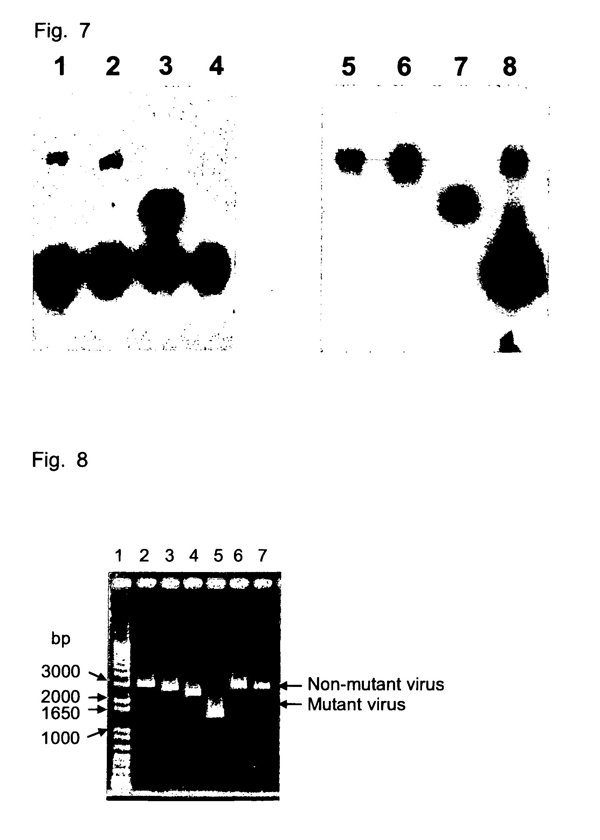

   Fig. 7 shows that a fusion protein derived from a gene of interest and a gp64 gene can be expressed even in a baculovirus incorporating a gp64 gene whose polynucleotide sequence has been modified so as not to alter the amino acid residues constituting GP64 protein.
[Fig. 8]
   Fig. 8 shows that no mutant virus is generated in the recombinant baculovirus shown in Fig. 7.

### [Modes for Carrying Out the Invention]

Abbreviations of amino acids, peptides, nucleotide sequences, nucleic acids, etc. as used herein conform to IUPAC-IUB regulations [IUPAC-IUB Communication on Biochemical Nomenclature, Eur. J. Biochem., 138: 9 (1984)], "Guideline for Preparing Specifications, etc. Including Nucleotide Sequences or Amino Acid Sequences" (ed. Japan Patent Office), and those conventionally used in the art.

As used herein, the term "DNA molecule" is intended to encompass double-stranded DNA and also single-stranded DNA (sense strand or antisense strand) which forms double-stranded DNA, and is not limited by its length. Therefore, unless otherwise specified, the polynucleotide (DNA molecule) of the present invention encoding a foreign immunogenic protein encompasses double-stranded DNA (including genomic DNA), single-stranded DNA (sense strand) (including cDNA), single-stranded DNA (antisense strand) having a sequence complementary to the sense strand, synthetic DNA, and fragments thereof.

As used herein, the term "polynucleotide" or "DNA molecule" is not defined by a functional region, and can include at least one of an expression suppression region, a coding region, a leader sequence, an exon, and an intron.
As used herein, "polynucleotide" includes RNA and DNA. A polypeptide having a specific amino acid sequence or a polynucleotide having a specific DNA sequence includes fragments, homologs, derivatives, and mutants thereof. Examples of the mutant DNA include naturally occurring allelic mutants, non-naturally occurring mutants, and mutants which have undergone deletion, substitution, addition, or insertion.

As used herein, the term "mutant virus DNA" may particularly refer to an unexpected PCR product which would otherwise be detected through analysis and in which a DNA region of a gene encoding a foreign immunogenic protein has been deleted, the PCR product being generated in a step of producing a recombinant virus of interest from a transfer vector by a homologous recombinant technique.

As used herein, "polynucleotide sequence is modified so as not to alter the amino acid residues constituting a protein" refers to the case where identity of a polynucleotide sequence is reduced so as not to alter the amino acid residues constituting a protein. This case can be done, for example, by suitably selecting codons encoding naturally occurring amino acid residues through visual means with reference to, for example, a codon table, or mechanically selecting by means of computer software, to thereby reduce identity in entire polynucleotide sequence with respect to the gene sequence for a naturally occurring virus-particle-forming protein.
Next will be described the transfer vector of the present invention, as well as the recombinant virus (in particular, recombinant baculovirus) of the present invention.

The transfer vector of the present invention is a plasmid which contains therein a fusion gene containing at least one gene encoding a virus-particle-forming protein and a gene encoding a foreign immunogenic protein, and which contains, on the upstream and downstream of the fusion gene, a DNA fragment capable of undergoing homologous recombination with baculovirus DNA. The transfer vector must contain, as a promoter, at least a baculovirus promoter capable of being expressed in insect cells. The transfer vector may contain a single promoter, or a dual promoter produced through ligation between a vertebrate promoter (mammalian promoter or avian promoter) and a baculovirus promoter.

Examples of the vertebrate promoter (i.e., promoter capable of functioning in vertebrate cells) include a mammalian promoter and an avian promoter. Examples of the mammalian promoter (i.e., promoter capable of functioning in mammalian cells) include a cytomegalovirus (CMV) promoter, an SV40 promoter, a retrovirus promoter, a metallothionein promoter, a heat-shock protein promoter, a CAG promoter, an elongation factor 1α promoter, an actin promoter, a ubiquitin promoter, an albumin promoter, and an MHC class promoter. Examples of the avian promoter include an actin promoter, a heat-shock protein promoter, an elongation factor promoter, a ubiquitin promoter, and an albumin promoter.

Examples of the baculovirus promoter include a polyhedrin (Polh) promoter, a p10 promoter, an IE1 promoter, a p35 promoter, a p39 promoter, and a gp64 promoter.

The dual promoter is preferably, for example, a promoter produced through ligation between a vertebrate promoter (e.g., a cytomegalovirus (CMV) promoter, a CAG promoter (prepared through improvement of the CMV promoter), or a ubiquitin (UBB) promoter bound to a CMV enhancer) and a baculovirus promoter (e.g., a polyhedrin (Polh) promoter).

When a vertebrate promoter (in particular, a mammalian promoter) is ligated to a baculovirus promoter, the polynucleotide sequences of these two promoters may be directly ligated to each other, or a DNA sequence may be provided between the DNA sequences of these promoters.
When a vertebrate promoter (in particular, a mammalian promoter) is ligated to a baculovirus promoter, any of these promoters may be provided closer to a gene to be expressed in a promoter region. In the Examples described hereinbelow, a dual promoter is prepared so that a baculovirus promoter is provided closer to a gene to be expressed than a mammalian promoter.

The sequence of a gene encoding a virus-particle-forming protein, which is included in the fusion gene DNA of the present invention, can be specified by a polynucleotide sequence in which nucleotides have been modified so as not to alter the amino acid residues constituting a corresponding naturally occurring virus-particle-forming protein; or a polynucleotide sequence in which nucleotides have been modified so that a portion of the amino acid residues constituting the naturally occurring virus-particle-forming protein is deleted (the polynucleotide sequence may further be modified so as not to alter the amino acid residues constituting the naturally occurring virus-particle-forming protein).

Modification of a polynucleotide sequence encoding a virus-particle-forming protein (including a partial polypeptide obtained through deletion of a portion of amino acid residues of the protein) so as not to alter the amino acid residues constituting a corresponding naturally occurring virus-particle-forming protein may be carried out by comparing the polynucleotide sequence including a virus DNA region encoding a protein capable of being a component of virus particles with the polynucleotide sequence for the naturally occurring virus-particle-forming protein. Modification is preferably carried out so that the identity in entire nucleotide sequence between the modified and unmodified polynucleotides is adjusted to 85% or less, more preferably 80% or less, even more preferably 70% or less, for determination of polynucleotide sequence information, from the viewpoints of suppression of generation of mutant virus DNA (which is generated through deletion of a DNA region of a gene encoding a foreign immunogenic protein) upon co-transfection, and production of a recombinant virus of high purity.
In the present invention, the identity between polynucleotide sequences may be determined through, for example, the Lipman-Pearson method (Science, 227, 1435, (1985)). For example, identity is calculated through analysis by use of a homology analysis (search homology) program of genetic information processing software Genetyx-Win (Ver. 5.1.1, Software Development Co., Ltd.), with ktup (unit size to compare) being set to 2.

When the virus-particle-forming protein is a baculovirus-forming protein (e.g., baculovirus GP64 protein), since a naturally occurring DNA sequence (endogenous virus DNA sequence) encoding the protein is present in the genomic DNA of the virus, polynucleotide sequence modification must be carried out through comparison with the polynucleotide sequence so that no alteration occurs in the amino acid residues constituting the naturally occurring virus-particle-forming protein, to thereby reduce identity in entire nucleotide sequence with respect to the gene sequence for the naturally occurring virus-particle-forming protein.

Modification of nucleotides in the gene sequence for the naturally occurring virus-particle-forming protein so as not to alter the amino acid sequence of the protein may be visually specified with reference to a known gene code, or may be specified, through use of software, etc., by the polynucleotide sequence of a gene whose expression in human cells (i.e., a subject to which a pharmaceutical composition is administered) is considered to be improved.

The polynucleotide sequence of a fusion gene containing the polynucleotide sequence obtained through modification of nucleotides in the gene sequence for the naturally occurring virus-particle-forming protein so as not to alter the amino acid sequence of the protein may be, for example, the polynucleotide sequence of SEQ ID NO: 34 shown in Example 1. The identity of this polynucleotide sequence is reduced to 77% or less after modification. In this case, production of mutant virus DNA through deletion of a DNA region of an immunogenic gene can be more reliably suppressed upon co-transfection, and a fusion protein of interest can be expressed on virus particles (see Examples 7, 8, and 10).

As used herein, "nucleotides are modified so that a portion of the amino acid residues constituting a virus-particle-forming protein is deleted" refers to modification of a polynucleotide sequence so as to produce a polypeptide (partial polypeptide) through deletion of a portion of the amino acid residues constituting the virus-particle-forming protein; for example, deletion of a portion of nucleotides of a naturally occurring polynucleotide encoding the virus-particle-forming protein. The thus-modified polynucleotide sequence may further be modified so as not to alter the amino acid residues constituting the partial polypeptide.

No particular limitation is imposed on the partial polypeptide, so long as it can be expressed as a protein capable of being a component of virus particles in insect cells. The partial polypeptide may be, for example, a polypeptide which is obtained through deletion of N-terminal amino acids of a virus-particle-forming protein and which has a length of, for example, 55 to 1%, preferably 35 to 1%, more preferably 15 to 1%, of that of the full-length amino acid sequence of the protein. The partial polypeptide is preferably a polypeptide shorter than the full-length sequence of a foreign immunogenic protein to which the polypeptide is fused.
Now will be described the case where the virus-particle-forming protein is baculovirus GP64 protein. As described in the Examples hereinbelow, in the case where the full-length amino acid sequence of the protein is 512 amino acid residues in length (including a signal sequence of 20 amino acid residues in length), and the amino acid sequence of a foreign immunogenic protein is 330 amino acid residues in length, when nucleotide modification is carried out so that the resultant fusion product contains a polypeptide of 261 amino acid residues in length (51%) or a polypeptide of 66 amino acid residues in length (13%), generation of mutant virus DNA can be considerably suppressed, and a DNA fusion product of intended high purity can be produced.
Therefore, when the virus-particle-forming protein is baculovirus GP64 protein, the partial polypeptide is, for example, a partial peptide obtained through deletion of 251 or 446 N-terminal amino acid residues from the amino acid residues (21 to 512) constituting the GP64 protein (exclusive of a signal sequence (amino acid residues 1 to 20)); more specifically, a partial peptide formed of amino acid residues 272 to 512 or amino acid residues 467 to 512.
The amino acid sequence of GP64 protein has a GenBank accession number of AAA66758 (SEQ ID NO: 62).

The gene encoding the virus-particle-forming protein is, for example, a gene encoding a protein capable of being a component of virus particles. Specific examples of the gene include genes for baculovirus GP64 protein (GenBank Accession No. L22858), vesicular stomatitis virus glycoprotein G (VSVG: GenBank Accession No. M27165), herpes simplex virus glycoprotein (KOS: GenBank Accession No. K01760), human immunodeficiency virus type 1 gp120 (GenBank Accession No. U47783), human respiratory syncytial virus membrane glycoprotein (GenBank Accession No. M86651), influenza A virus hemagglutinin protein (GenBank Accession No. U38242), etc.; and genes for envelope proteins of viruses closely related to the baculovirus. Of these, a gene for baculovirus GP64 protein is preferred.

The polynucleotide of the aforementioned gene encoding the virus-particle-forming protein can be readily produced and obtained through, for example, synthesis (chemical DNA synthesis) on the basis of the nucleic acid information of a polynucleotide obtained through modification of the polynucleotide sequence of a gene encoding the amino acid sequence of a corresponding naturally occurring virus-particle-forming protein so as not to alter the amino acid residues constituting the naturally occurring virus-particle-forming protein, or a polynucleotide encoding a partial polypeptide of the naturally occurring virus-particle-forming protein obtained through deletion of a portion of the amino acid residues constituting the naturally occurring virus-particle-forming protein (including a polynucleotide obtained through modification of the polynucleotide sequence so as not to alter the amino acid residues constituting the partial polypeptide).

The polynucleotide sequence of a gene encoding a foreign immunogenic protein, which is included in the DNA of the fusion gene of the present invention, can be specified by the naturally occurring nucleic acid information of a polynucleotide sequence encoding the foreign immunogenic protein. However, similar to the case of the aforementioned virus-particle-forming protein, the polynucleotide may be a polynucleotide whose sequence has been modified so as not to alter the amino acid residues constituting a corresponding naturally occurring foreign immunogenic protein, and the polynucleotide can be readily produced and obtained through, for example, synthesis (chemical DNA synthesis) on the basis of the nucleic acid information of the polynucleotide sequence.

The polynucleotide sequence of a gene encoding the amino acid sequence of a foreign immunogenic protein, or modification of nucleotides in the polynucleotide sequence so as not to alter the amino acid residues constituting the foreign immunogenic protein may be visually specified with reference to a known gene code, or may be specified, through use of software (Gene Designer (DNA 2.0: Gene Designer: https://www.dna20.com/index.php?pageID=220) or Optimizer (http://genomes.urv.es/OPTIMIZER)), etc., by the polynucleotide sequence of a gene whose expression in vertebrate cells (specifically, mammalian cells; in particular, human cells) is considered to be improved.

In the present invention, the foreign-immunogenic-protein-encoding gene may be, for example, a gene encoding an antigen protein of, for example, an infection in a mammal such as human, cattle, horse, pig, sheep, monkey, mouse, dog, or cat; or a gene for an antigen of, for example, an infection in an avian such as chicken, duck, pigeon, turkey, guinea fowl, or parrot (e.g., avian influenza S antigen). As used herein, "foreign" gene refers to a gene transferred into a cell from outside thereof (even if the same gene is present in the cell).
Pathogen genes which have been reported to be associated with infections in the aforementioned mammals and avians are readily available from organizations having public databases (e.g., GenBank) in which pathogen genes are registered. Specific examples of the gene encoding an antigen protein associated with an infection, etc. in a mammal include genes encoding antigen proteins serving as immunogens in immunotherapy (including vaccine therapy) for the prevention or treatment of infections (e.g., malaria, influenza, and tuberculosis), autoimmune diseases, cancer, etc.

Examples of the gene encoding the amino acid sequence of a malaria antigen include genes encoding proteins such as CSP protein, MSP1 protein, LSA1 protein, SERA protein, TRAMP protein, and AMA1 protein.

Examples of the gene encoding the amino acid sequence of an influenza virus antigen include genes encoding proteins such as HA antigen (hemagglutinin antigen), NA antigen (neuraminidase antigen), M2 antigen (matrix protein antigen), and NP antigen (nucleoprotein antigen).

Examples of the gene encoding the amino acid sequence of an antigen protein for tuberculosis include genes encoding proteins such as HSP65 (65-kDa heat-shock protein), α antigens (Antigen 85A, Antigen 85B, and Antigen 85C), Mtb72f, MDP-1, ESAT-6, MPB51, Mtb8.8, Mtb9.9, Mtb32, Mtb39, and Mtb11.

Examples of the gene encoding the amino acid sequence of an antigen protein for cancer include genes encoding proteins such as CEA, WT1, MAGE, HER2/neu, and SART.

In the present invention, no particular limitation is imposed on the foreign-immunogenic-protein-encoding gene, so long as the gene is transferred from outside. The foreign-immunogenic-protein-encoding gene may encompass, in addition to genes encoding the aforementioned immunogens present outside of the human body, cytokine genes present inside of the human body (e.g., an IL-12 gene, an IL-6 gene, an IL-6 receptor gene, an IL-2 gene, an IL-18 gene, an IFN-γ gene, and an M-CSF gene), and fusion genes obtained by fusing any antigen having immunogenicity with any of the aforementioned antigen protein genes through a genetic recombination technique.

The polynucleotide sequence employed in the present invention is not limited by the full-length sequence of a polynucleotide sequence encoding a polypeptide of any of the aforementioned antigen proteins having immunogenicity. So long as a protein having an amino acid sequence encoded by the polynucleotide sequence exhibits antigenicity, the polynucleotide sequence may be a polynucleotide sequence encoding a partial amino acid sequence or a repeated partial amino acid sequence, or a polynucleotide sequence encoding a partial amino acid sequence obtained through fusion between partial amino acid sequences of a protein.

The naturally occurring polynucleotide sequence of a gene encoding any of the aforementioned foreign immunogenic proteins, or a polynucleotide sequence which has been modified so as not to alter the amino acid residues constituting the foreign immunogenic protein may be readily produced and obtained by synthesizing a DNA fragment corresponding to the polynucleotide sequence (chemical DNA synthesis). Production of the polynucleotide sequence may employ a common genetic engineering technique [see, for example, Molecular Cloning 2nd Ed., Cold Spring Harbor Lab. Press (1989); or Zoku Seikagaku Jikken Koza "Idenshi Kenkyuho I, II, III" edited by the Japanese Biochemical Society (1986)].

Examples of the method for synthesizing the polynucleotide include chemical synthesis means, such as the phosphate triester method and the phosphate amidite method [J. Am. Chem. Soc., 89, 4801 (1967); J. Am. Chem. Soc., 91, 3350 (1969); Science, 150, 178 (1968); Tetrahedron Lett., 22, 1859 (1981); and Tetrahedron Lett., 24, 245 (1983)], and combinations of these methods. More specifically, the polynucleotide may be chemically synthesized through the phosphoramidite method or the triester method, or may be synthesized by means of a commercially available automated oligonucleotide synthesizer. A double-stranded fragment may be obtained by synthesizing a complementary strand and annealing the complementary strand with a chemically synthesized single strand under appropriate conditions, or adding the complementary strand and an appropriate primer sequence to the chemically synthesized single strand by use of DNA polymerase.

The polynucleotide of a gene encoding a naturally occurring foreign immunogenic protein may be produced through a genetic engineering technique. More specifically, production of the polynucleotide may be carried out by preparing a cDNA library, through a customary method, from an appropriate origin in which the polynucleotide of the gene is expressed, and by selecting a clone of interest from the cDNA library by use of an appropriate probe corresponding to the DNA of the gene or an antibody to an expression product of the gene [see, for example, Proc. Natl. Acad. Sci., USA., 78, 6613 (1981); or Science, 222, 778 (1983)].

Examples of the aforementioned origin of genomic DNA include cells and tissue in which the DNA of the foreign-immunogenic-protein-encoding gene is expressed; and cultured cells derived therefrom. The origin is particularly preferably, for example, an extract of cells infected with influenza virus, an extract of erythrocytes infected with plasmodium, or an extract of *Mycobacterium tuberculosis.* Extraction and separation of total DNA or RNA from such an origin, separation and purification of mRNA, obtainment and cloning of cDNA, or a similar process may be carried out through a customary method.

As described above, production of the DNA of the foreign-immunogenic-protein-encoding gene may be carried out by use of the cDNA library of any of the aforementioned immunogens (i.e., any of the aforementioned extracts) obtained through extraction, separation, and purification of mRNA from the tissue or cells of the immunogen. Alternatively, production of the DNA may be carried out by use of a phage library prepared through the following procedure: mRNA is extracted from any of the aforementioned immunogens; poly A is added to the RNA; the poly A-added RNA is collected; cDNA is produced by use of a reverse transcriptase; restriction enzyme sites are added to both ends of the cDNA; and the cDNA is incorporated into a phage.

No particular limitation is imposed on the method for screening, from the cDNA library, the DNA of the foreign-immunogenic-protein-encoding gene, and this screening may be carried out through a customary method. Specific examples of the screening method include a method of selecting a corresponding cDNA clone through immunological screening employing a specific antibody (e.g., anti-malaria antibody, anti-influenza virus antibody, or anti-*Mycobacterium tuberculosis* antibody) against a protein produced by the cDNA; a plaque hybridization method employing a probe which selectively binds to a DNA sequence of interest; a colony hybridization method; and combinations thereof.

The probe employed in any of the aforementioned hybridization methods is generally, for example, a DNA fragment chemically synthesized on the basis of the DNA sequence information of the foreign-immunogenic-protein-encoding gene. The DNA sequence of the already obtained foreign-immunogenic-protein-encoding gene or a fragment thereof may be advantageously employed as the aforementioned probe. Furthermore, the probe employed for the aforementioned screening may be a sense primer or antisense primer designed on the basis of the DNA sequence information of the foreign immunogenic gene.

The DNA fragment (nucleotides) employed as the probe is a partial DNA fragment (nucleotides) corresponding to the DNA sequence of the foreign immunogenic gene, and has at least 15 consecutive nucleotides, preferably at least 20 consecutive nucleotides, more preferably at least 30 consecutive nucleotides. A positive clone itself for producing the aforementioned DNA may also be employed as the probe.

For preparation of the DNA of the foreign-immunogenic-protein-encoding gene, the DNA/RNA amplification method through PCR [Science, 230, 1350 (1985)] may be suitably employed. Particularly when full-length cDNA is difficult to obtain from the library, for example, the RACE method [Rapid amplification of cDNA ends; Jikken Igaku, 12 (6), 35 (1994)], in particular, the 5'-RACE method [M. A. Frohman, et al., Proc. Natl. Acad. Sci., USA., 8, 8998 (1988)] is suitably employed.

A primer employed for PCR may be appropriately designed on the basis of the DNA sequence information of the foreign-immunogenic-protein-encoding gene, and synthesized through a customary method. As described in the Examples hereinbelow, the primer employed may be DNA portions (SP6 promoter primer and T7 terminator primer) added to both ends of the DNA of the foreign-immunogenic-protein-encoding gene incorporated into a vector plasmid.

Isolation/purification of the DNA fragment amplified through PCR may be carried out through a customary method (e.g., gel electrophoresis).
The thus-obtained DNA or DNA fragments of the foreign-immunogenic-protein-encoding gene may be sequenced through a customary method; for example, the dideoxy method [Proc. Natl. Acad. Sci., USA., 74, 5463 (1977)] or the Maxam-Gilbert method [Methods in Enzymology, 65, 499 (1980)], or conveniently by means of a commercially available sequencing kit.

The polynucleotide sequence of the fusion gene of the present invention may be a polynucleotide sequence formed by directly ligating the two genes of the fusion genes to each other. Alternatively, a DNA fragment may be provided between these two genes (in this case, the downstream gene and the upstream gene must be located so as not to cause frameshifting). Preferably, the antigen-presenting domain of a foreign immunogenic protein of interest is fused with a virus-particle-forming protein. Therefore, the foreign immunogenic protein of interest must be employed in the form of being fused with the virus-particle-forming protein (i.e., without separation of these proteins from each other). In the recombinant virus exemplified in this embodiment, the N terminus of baculovirus GP64 protein is exposed to the outside of a virus particle, and the C terminus thereof is exposed to the inside of the particle. Therefore, when a foreign immunogenic protein of interest is fused with the N terminus of the GP64 protein, the entirety of the foreign immunogenic protein is exposed, as a component of virus particles, to the outside of the particles in insect cells. This structure further facilitates antigen presentation, and meets the requirement for a vaccine product.

Preferably, the fusion gene of the present invention, which includes at least one gene encoding a virus-particle-forming protein and a gene encoding a foreign immunogenic protein, includes, upstream thereof, a polynucleotide encoding a signal peptide (signal sequence) which promotes secretion of an expressed protein to the outside of a host.
The signal sequence is preferably a signal sequence of the virus-particle-forming protein to be expressed, but may be a signal sequence of a protein capable of functioning in cells in which virus replication can occur.
When the signal sequence is derived from the virus-particle-forming protein to be expressed, the fusion gene of the present invention may have a structure in which the polynucleotide sequence of a gene encoding a foreign immunogenic protein is provided (inserted) between the polynucleotide encoding the signal sequence and the polynucleotide sequence of the gene encoding the virus-particle-forming protein. Specifically, the fusion gene of the present invention preferably has a structure in which the polynucleotide sequence of the gene encoding the foreign immunogenic protein is inserted between the polynucleotide encoding the signal sequence of the virus-particle-forming protein (including the signal sequence) and the polynucleotide encoding the virus-particle-forming protein, wherein nucleotides of a naturally occurring polynucleotide sequence encoding the full-length amino acid sequence of the virus-particle-forming protein (including the signal sequence) have been modified so as not to alter the amino acid residues constituting a corresponding naturally occurring virus-particle-forming protein.
In other words, the transfer vector of the present invention encompasses a transfer vector containing a DNA region of a gene encoding a foreign immunogenic protein inserted between virus DNA regions including at least one gene encoding a virus-particle-forming protein, wherein the polynucleotide sequence of the N-terminal and/or C-terminal virus DNA region fused with the DNA region of the foreign-immunogenic-protein-encoding gene is modified so that the polynucleotide sequence exhibits reduced identity to a naturally occurring polynucleotide sequence of the same virus DNA region(s) and/or so that a portion of the amino acid residues constituting a corresponding naturally occurring virus-particle-forming protein is deleted.

For incorporation of the aforementioned fusion gene into the transfer vector, the polynucleotide sequence of the fusion gene of the present invention may be formed in advance, followed by incorporation of the polynucleotide sequence into the vector. Alternatively, the polynucleotide sequence of one of the genes of the fusion gene may be incorporated into the vector, followed by incorporation of the polynucleotide sequence of the other gene into the vector, to thereby form the fusion gene in the vector.

In the transfer vector of the present invention, the aforementioned fusion gene is ligated to the downstream of a single baculovirus promoter, or a dual promoter prepared through ligation between a vertebrate promoter (in particular, a mammalian promoter) and a baculovirus promoter.
The transfer vector of the present invention may be produced through the following procedure: a commercially available expression vector containing a promoter region (i.e., a component of the transfer vector; for example, a vertebrate promoter (in particular, a mammalian promoter) or a baculovirus promoter) and a polynucleotide sequence encoding a virus-particle-forming protein is provided; and the polynucleotide sequence of a fusion gene is inserted into the cloning region of the vector through, for example, optional cleavage by use of a restriction enzyme or incorporation of another promoter, the fusion gene containing a gene encoding a foreign immunogenic protein of interest and the aforementioned modified gene encoding the virus-particle-forming protein of the present invention (i.e., a polynucleotide whose sequence has been modified so as not to alter the amino acid residues constituting a corresponding naturally occurring virus-particle-forming protein; or a polynucleotide encoding a partial polypeptide obtained through deletion of a portion of the amino acid residues constituting the naturally occurring virus-particle-forming protein (including a polynucleotide whose sequence has been modified so as not to alter the amino acid residues constituting the partial polypeptide)). Alternatively, the modified gene of the present invention encoding the virus-particle-forming protein may be incorporated into a plasmid in advance, and the gene encoding the foreign immunogenic protein of interest may be ligated to the modified gene. The polynucleotide sequence encoding a polypeptide of any of the aforementioned antigen proteins having immunogenicity is not limited by the full-length sequence of the polynucleotide sequence. So long as a protein having an amino acid sequence encoded by the polynucleotide sequence exhibits antigenicity, the polynucleotide sequence may be a polynucleotide sequence encoding a partial amino acid sequence or a repeated partial amino acid sequence, or a polynucleotide sequence encoding a partial amino acid sequence obtained through fusion between partial amino acid sequences of a protein.

For ligation of the above-modified gene encoding the virus-particle-forming protein to the aforementioned foreign-immunogenic-protein-encoding gene (including a polynucleotide whose sequence has been modified so as not to alter the amino acid residues constituting the foreign immunogenic protein), a necessary component may be incorporated by, for example, inserting the foreign-immunogenic-protein-encoding gene into the N-terminal region of the modified gene encoding the virus-particle-forming protein.
In the present invention, a plasmid vector having a structure that can express a gene encoding a foreign immunogenic protein of interest as an antigen protein in both insect cells and vertebrate cells (in particular, mammalian cells) may be produced by use of a commercially available plasmid having a structure that satisfies a portion of the requirement for the plasmid vector.
The amino acid sequence of a peptide may be provided for cleaving the fusion protein by an enzyme in vertebrate cells.

In the transfer vector of the present invention, an enhancer for increasing transcription activity in vertebrate cells (in particular, mammalian cells) may be provided on the upstream of the promoter region. For termination of transcription, a vertebrate terminator region (e.g., a rabbit β-globulin terminator) which is effective in vertebrate cells may be provided on the downstream of a gene to be fused and expressed.

An example of the transfer vector of the present invention is a transfer vector having a structure including a CAG promoter (i.e., a vertebrate promoter; in particular, a mammalian promoter) ligated to a polyhedrin (Polh) promoter (i.e., a baculovirus promoter), and, on the downstream thereof, a fusion gene containing an influenza virus antigen gene or a falciparum malaria antigen gene (the polynucleotide sequence thereof may be modified so as not to alter the amino acid residues constituting a corresponding naturally occurring foreign immunogenic protein) and a gp64 gene whose polynucleotide sequence has been modified so as not to alter the amino acid residues constituting gp64 or a gene encoding a partial polypeptide of gp64 obtained through deletion of a portion of the amino acid residues constituting gp64 (including a gene whose polynucleotide sequence has been modified so as not to alter the amino acid residues constituting the partial polypeptide of gp64). Specific examples include pCAP-HA1/Vietnam/51, pCAP-HA1/Vietnam/101, pCAP-HA1/Vietnam/154, pCAP-HA1/Vietnam/201, pCAP-HA1/Vietnam/272, pCAP-HA1/Vietnam/467, pCAP-HA1/Anhui, pCAP-HA1/Anhui/272, pCAP-HA1/Anhui/467, pCAP-HA1/Vietnam/OG, pCAP-CO/full/OG, pCAP-CO/76/OG, pCAP-CO/205/OG, and pCAP-CO/76/467. The nucleotide sequences of particularly preferred transfer vectors of these examples are described below (Table 1).

**[Table 1]**

| Transfer vector | Nucleotide sequence/(size bp) | | |
|---|---|---|---|
| | Signal peptide | Foreign immunogenic protein | Virus-particle-forming protein |
| pCAP-HA1/Vietnam/272 | GP64 (1-20) 60 bp SEQ ID NO: 53 | HA/Vet (17-346) 990 bp SEQ ID NO: 54 | GP64 (272-512) 723 bp SEQ ID NO: 59 |
| pCAP-HA1/Vietnam/467 | GP64 (1-20) 60 bp SEQ ID NO: 53 | HA/Vet (17-346) 990 bp SEQ ID NO: 54 | GP64 (467-512) 138 bp SEQ ID NO: 60 |
| pCAP-HA1/Vietnam/OG | GP64 (1-20) 60 bp SEQ ID NO: 53 | HA/Vet (17-346) 990 bp SEQ ID NO: 54 | GP64 (21-512) 1476 bp SEQ ID NO: 61 |
| pCAP-CO/full/OG | GP64 (1-20) 60 bp SEQ ID NO: 53 | PfCSP (1-397) 1191 bp SEQ ID NO: 55 | GP64 (21-512) 1476 bp SEQ ID NO: 61 |
| pCAP-CO/76/OG | GP64 (1-20) 60 bp SEQ ID NO: 53 | PfCSP (76-373) 894 bp SEQ ID NO: 56 | GP64 (21-512) 1476 bp SEQ ID NO: 61 |
| pCAP-CO/205/OG | GP64 (1-20) 60 bp SEQ ID NO: 53 | PfCSP (205-373) 507 bp SEQ ID NO: 57 | GP64 (21-512) 1476 bp SEQ ID NO: 61 |
| pCAP-CO/76/467 | GP64 (1-20) 60 bp SEQ ID NO: 53 | PfCSP (76-373) 894 bp SEQ ID NO: 56 | GP64 (467-512) 138 bp SEQ ID NO: 60 |
| pCAP-HA1/Anhui/272 | GP64 (1-20) 60 bp SEQ ID NO: 53 | HA/Anh (17-345) 987 bp SEQ ID NO: 58 | GP64 (272-512) 723 bp SEQ ID NO: 59 |
| pCAP-HA1/Anhui/467 | GP64 (1-20) 60 bp SEQ ID NO: 53 | HA/Anh (17-345) 987 bp SEQ ID NO: 58 | GP64 (467-512) 138 bp SEQ ID NO: 60 |
| pCAP-HA1/Anhui/OG | GP64 (1-20) 60 bp SEQ ID NO: 53 | HA/Anh (17-345) 987 bp SEQ ID NO: 58 | GP64 (21-512) 1476 bp SEQ ID NO: 61 |

Thus, the transfer vector of the present invention can be produced. An example of a production process therefor includes the following steps (a) to (c) and/or (d) and (e):
(a) a step of modifying the polynucleotide sequence of a gene encoding a virus-particle-forming protein so as not to alter the amino acid residues constituting a corresponding naturally occurring virus-particle-forming protein, and obtaining the nucleic acid sequence information of a polynucleotide including the gene encoding the virus-particle-forming protein;
(b) a step of synthesizing the polynucleotide on the basis of the nucleic acid sequence information obtained in step (a);
(c) a step of ligating or inserting, into the polynucleotide synthesized in step (b), a polynucleotide of a gene encoding a foreign immunogenic protein;
(d) a step of synthesizing a polynucleotide encoding a partial polypeptide of the naturally occurring virus-particle-forming protein obtained through deletion of a portion of the amino acid residues constituting the naturally occurring virus-particle-forming protein, or a polynucleotide whose sequence has been modified so as not to alter the amino acid residues constituting the partial polypeptide; and
(e) a step of ligating or inserting, into the polynucleotide synthesized in step (d), a polynucleotide of the gene encoding the foreign immunogenic protein.

The recombinant baculovirus of the present invention may be produced by co-transfecting the aforementioned transfer vector and baculovirus DNA into a host cell.

Baculoviruses are entomopathogenic viruses that infect insects and a group of DNA viruses (baculovirus family) having circular double-stranded DNA as a gene. Among baculoviruses, a group of viruses called "nucleopolyhedrovirus (NPV)" produce an inclusion body called "polyhedron" in the nucleus of infected cells at a late stage of infection. Even when a foreign gene to be expressed is inserted into cells instead of a polyhedron gene, such a baculovirus infects the cells, proliferates therein, and produces a large amount of a foreign immunogenic gene product of interest without causing any problem. Therefore, baculoviruses have been recently applied to the production of a protein of interest.

Examples of such a baculovirus include *Autographa californica* multiple nucleopolyhedrovirus (AcNPV), *Bombyx mori* nucleopolyhedrovirus (BmNPV), *Orgyia pseudotsugata* multiple nucleopolyhedrovirus (OpNPV), and *Lymantria disper* multiple nucleopolyhedrovirus (LdNPV).

No particular limitation is imposed on the baculovirus DNA employed in the present invention, so long as it can undergo homologous recombination with the transfer vector of the present invention. The baculovirus DNA may be wild-type, mutant, or recombinant baculovirus DNA.
The baculovirus DNA employed may be a commercially available product; for example, a product obtained through deletion of a polyhedrin gene from AcNPV, such as BacVector-1000 DNA or BacVector-2000 DNA (product of Novagen).

For induction of homologous recombination, preferably, the transfer vector and baculovirus DNA are mixed at a ratio by weight of, for example, about 1 : 1 to about 10 : 1.

No particular limitation is imposed on the method for incorporating the transfer vector into a host cell, as well as the method for transforming the host cell with the vector, and various general and conventional methods well known in the art may be employed. Incorporation or transformation may be carried out through, for example, a common genetic recombination technique (see, for example, Science, 224, 1431 (1984); Biochem. Biophys. Res. Comm., 130, 692 (1985); or Proc. Natl. Acad. Sci. USA, 80, 5990 (1983)). Expression of the transfer vector may be carried out through the method of Ohno et al., "Tanpaku Jikken Protocol 1 Kino Kaiseki-hen, Saibo Kogaku Bessatsu, Jikken Protocol Series, 1997, Shujunsha." General techniques including handling of insect cells, genetic recombination, and co-transfection may be those similar to well-known methods for preparing a recombinant virus in insect cells (Yoshiharu Matsuura, Tanpaku Kakusan Koso, 37, 211-222 (1992), and Yoshiharu Matsuura, Saibo, 33 (2) 30-34 (2001)).
Co-transfection may be carried out by means of a commercially available vector transfection kit (BacVector Transfection Kits, product of Novagen) according to the manual attached to the vector transfection kit.

More specifically, the above-produced transfer vector and baculovirus DNA which undergoes homologous recombination therewith are co-transfected into a host cell; for example, insect cells such as Sf9 cells or Sf21 cells derived from *Spodoptera frugiperda,* or Tn5 cells derived from *Trichoplusia* ni (High Five cells, product of Invitrogen).

After co-transfection of the transfer vector and baculovirus DNA into insect cells and subsequent culturing, plaques of the resultant recombinant virus are prepared from the culture supernatant and suspended in an agar culture medium, and the virus is eluted from the agar medium by vortex, followed by centrifugation, to thereby produce a solution containing the recombinant virus.

For example, according to the aforementioned recombinant baculovirus production method, baculovirus DNA and a transfer vector (e.g., pCAP-HA1/Vietnam, pCAP-HA1/Vietnam/51, pCAP-HA1/Vietnam/101, pCAP-HA1/Vietnam/154, pCAP-HA1/Vietnam/201, pCAP-HA1/Vietnam/272, pCAP-HA1/Vietnam/467, pCAP-HA1/Anhui, pCAP-HA1/Anhui/272, pCAP-HA1/Anhui/467, pCAP-HA1/Vietnam/OG, pCAP-CO/full/OG, pCAP-CO/76/OG, pCAP-CO/205/OG, or pCAP-CO/76/467) may be co-transfected into Sf9 insect cells, to thereby produce a recombinant baculovirus of AcNPV-CAP-HA1/Vietnam, AcNPV-CAP-HA1/Vietnam/51, AcNPV-CAP-HA1/Vietnam/101, AcNPV-CAP-HA1/Vietnam/154, AcNPV-CAP-HA1/Vietnam/201, AcNPV-CAP-HA1/Vietnam/272, AcNPV-CAP-HA1/Vietnam/467, AcNPV-CAP-HA1/Anhui, AcNPV-CAP-HA1/Anhui/272, AcNPV-CAP-HA1/Anhui/467, AcNPV-CAP-HA1/Vietnam/OG, AcNPV-CAP-CO/full/OG, AcNPV-CAP-CO/76/OG, AcNPV-CAP-CO/205/OG, or AcNPV-CAP-CO/76/467.

In addition to the aforementioned method, there may be employed a recombinant baculovirus production method in which a foreign gene is effectively inserted, in *Escherichia coli* cells, into a phagemid (bacmid) incorporating the entire baculovirus genome by use of a transposon. According to this method, a recombinant baculovirus can be readily produced and collected by only extracting a bacmid containing a virus gene from microbial cells, and transfecting the bacmid in insect cells.

Thus, the above-produced transfer vector and baculovirus DNA may be co-transfected into insect cells such as Sf9 cells, to thereby produce a recombinant baculovirus.
The thus-produced recombinant baculovirus may be purified through any known virus purification method. For example, a virus stock (generally 1 × 10⁷⁻⁸ pfu/mL) produced through the aforementioned recombinant baculovirus production method is inoculated (0.5 to 1.0 mL) into insect cells such as Sf9 cells (1 × 10⁷ cells/10cm-dish), and, several days (4 days) after infection, the resultant culture supernatant is collected. Thereafter, virus pellets obtained through centrifugation (25,000 rpm, 60 minutes, 4°C) are suspended in a buffer such as PBS. The resultant suspension is subjected to 10 to 60% sucrose gradient centrifugation, and the resultant virus band is collected. The thus-collected product is further suspended in PBS, followed by centrifugation. The resultant purified recombinant virus pellets are stored in a buffer such as PBS at 4°C.
The infectivity titer of the above-produced purified recombinant baculovirus may be determined through a plaque assay (Fields VIROLOGY 4th Edition pp. 29-32, 2001) by use of insect cells such as Sf9 cells.

The thus-produced recombinant baculovirus may be cultured through a customary method. Through this culturing, a fusion product of a foreign immunogenic protein and a virus-particle-forming protein (i.e., expression product of the fusion gene) is produced (accumulated or secreted) inside or outside of insect cells, or on the cell membrane.

The medium employed for culturing may be appropriately selected, depending on the type of the host cell employed, from among various conventionally used media. Culturing may be carried out under conditions suitable for growth of the host cell.
The present invention will next be described in more detail by way of examples, which are given only for the illustration purpose and should not be construed as limiting the invention thereto.

### [Examples]

### Example 1: Transfer vector plasmid and production method therefor

### (1) Production of transfer vector plasmid pBACsurf-HA1/PR8

### (1.1) Construction of plasmid pBACsurf-Hsp65

PCR was performed by use of the genomic DNA of tubercule virus H37Rv as a template, primer phsp65-F1 (5' - AATAATAGATCTAATGGCCAAGACAATTGCGTACGACGAAGA-3' (SEQ ID NO: 1): *Bgl*II site underlined), and primer phsp65-R1 (5' - AATCCAATGCGGCCGCGGGAATTCGATTCCTGCAGGTCAGAAATCCATGCCACCCATGTCG CC-3' (SEQ ID NO: 2) ; NotI underlined).
After purification, the PCR product of interest was digested with restriction enzymes *Bgl*II and NotI, and the obtained fragment was inserted into pcDNA3.1(+) (product of Invitrogen) which had been digested with restriction enzymes *Bam*HI and *Not*I, to thereby construct plasmid pcDNA-Ighsp65. PCR was further performed by use of pcDNA-Ighsp65 as a template, primer phsp65-F2 (5' - CACCCCTGCAGG [ACTACAAGGACGACGATGACAAG] GAATTCATGGCCAAGACAATTGCG TACGACGAAGAGGCC-3' (SEQ ID NO: 3): *Sse*8387I site and *Eco*RI site underlined, and flag sequence enclosed with [ ]), and primer phsp65-R2 (5' -CCCGGGCGAAATCCATGCCACCCATGTCGCCGCCACC-3' (SEQ ID NO: 4): *Xma*I site underlined), and the obtained Hsp65 gene DNA fragment was cloned into pENTR/D-TOPO (product of Invitrogen). The clone was digested with restriction enzymes *Sse*8387I and *Xma*I, and the fragment was inserted into pBACsurf-CSP (Yoshida et al. Virology 316: 161-70, 2003) which had been digested with restriction enzymes PstI and *Xma*I, to thereby construct plasmid pBACsurf-Hsp65.

### (1.2) Construction of plasmid pBACsurf-HA1/PR8

From a supernatant of MDCK cells infected with influenza virus PR8/34, RNA was extracted by means of QIAamp MiniElute Virus Spin Kit (product of QIAGEN). RT-PCR was performed by use of the obtained RNA, primer HA-f (5' - CCTGCAGGTATGAAGGCAAACCTACTGGTC -3' (SEQ ID NO: 5): *Sbf*I site underlined), and primer HA-r (5' -GCCCGGGCGATGCATATTCTGCA-3' (SEQ ID NO: 6): SbfI site underlined). The influenza virus HA gene fragment was cloned into pCR-Blunt II-TOPO (product of Invitrogen) . The thus-obtained plasmid was denominated pCR-Blunt-HA. PCR was performed by use of pCR-Blunt-HA as a template, primer pHA-F1 (5'-CACCGAATTCGACACAATATGTATAGGCTACCATGCG-3' (SEQ ID NO: 7): *EcoR*I site underlined), and primer pHA-R1 (5' - CCCGGGCACCTCTGGATTGGATGGACGGAATG-3' (SEQ ID NO: 8): *Xma*I site underlined). The obtained H1N1/HA1 gene DNA fragment was digested with restriction enzymes *Eco*RI and *Xma*I, and the fragment was inserted into pBACsurf-Hsp65 which had been treated with restriction enzymes *Eco*RI and *Xma*I, to thereby construct plasmid pBACsurf-HA1/PR8.

### (2) Production of transfer vector plasmid pCAP-HA1/PR8

PCR was performed by use of pBACsurf-HA1/PR8 as a template, Polh-f *Rsr*II (5' - GGGCGGACCGGATAATTAAAATGATAACCATCTCG-3' (SEQ ID NO: 9): *Rsr*II site underlined), and GP64-r DraIII (5' - GGGCACTTAGTGATATTGTCTATTACGGTTTCTAATC-3' (SEQ ID NO: 10): DraIII site underelined). The obtained DNA fragment was treated with restriction enzymes *Rsr*II and DraIII, and pTriEx-1.1 (product of Novagen) was inserted into the vector which had been digested with restriction enzymes *Rsr*II and DraIII, to thereby construct plasmid pCAP-HA1/PR8.

### (3) Construction of transfer vector plasmids pCAP-HA1/Vietnam, pCAP-HA1/Vietnam/51, pCAP-HA1/Vietnam/101, pCAP-HA1/Vietnam/154, pCAP-HA1/Vietnam/201, pCAP-HA1/Vietnam/272, and pCAP-HA1/Vietnam/467

From an amino acid sequence of the HA1 region of hemagglutinin of influenza virus H5N1/Vietnam, an artificial gene sequence (SEQ ID NO: 11) was synthesized. PCR was performed by use of the artificial sequence as a template, VN-F1 (5' -CAGTCTGCAGGACCAGATCTGTATC-3' (SEQ ID NO: 12): PstI site underlined), and VN-R4 (5' -CAGTCCCGGGCTCTCTTCTTCCTGC-3' (SEQ ID NO: 13): *Xma*I site underlined). A fragment digested from the PCR product with restriction enzymes PstI and Xmal was inserted into pCAP-HA1/PR8 which had been treated with restriction enzymes PstI and Xmal. The thus-constructed plasmid was denominated pCAP-HA1/Vietnam. PCR was further performed by use of pCAP-HA1/PR8 as a template, GP64-r DraIII (5'-GGGCACTTAGTGATATTGTCTATTACGGTTTCTAATC-3' (SEQ ID NO: 20) :*Dra*III site underlined), and gp64 (51) -f (5' - GACTCCCCGGGTGGAAATCACCATCGTGGAGACG-3' (SEQ ID NO: 14): Xmal site underlined), gp64(101)-f (5' - GACTCCCCGGGATTTGCTTATGTGGAGCATCAGG-3' (SEQ ID NO: 15): Xmal site underlined), gp64(154)-f (5' - GACTCCCCGGGCGCACCACACGTGCAACAAATCG-3' (SEQ ID NO: 16): Xmal site underlined), gp64 (201) -f (5' - GACTCCCCGGGACACTGTGCTTCATCGAGACGGC-3' (SEQ ID NO: 17): Xmal site underlined), gp64 (272) -f (5' - GACTCCCCGGGTCGAGCACCGAGTCAAGAAG-3' (SEQ ID NO: 18): Xmal site underlined), or gp64(467)-f (5' - GACTCCCCGGGACATCACTTCCATGGCTGAA-3' (SEQ ID NO: 19): Xmal site underlined). Each of the obtained DNA fragments was digested with restriction enzymes *Xma*l and *Dra*III, and the product was inserted into pCAP-HA1/Vietnam which had been treated with restriction enzymes *Xmal* and *Dra*III. The thus-constructed plasmids were denominated pCAP-HA1/Vietnam/51, pCAP-HA1/Vietnam/101, pCAP-HA1/Vietnam/154, pCAP-HA1/Vietnam/201, pCAP-HA1/Vietnam/272, and pCAP-HA1/Vietnam/467, respectively.
Note that the GenBank accession number of the amino acid sequence of hemagglutinin of influenza virus A/Vietnam/1203/2004 (H5N1) is AAW80717.

### (4) Construction of transfer vector plasmids pCAP-HA1/Anhui, pCAP-HA1/Anhui/272, and pCAP-HA1/Anhui/467

From an amino acid sequence of the HA1 region of hemagglutinin of influenza virus H5N1/Anhui/1/05, an artificial gene sequence (SEQ ID NO: 21) was synthesized. PCR was performed by use of the artificial sequence as a template, AH-F1 (5'-CAGTCTGCAGGACCAGATTTGCATC-3' (SEQ ID NO: 22): PstI site underlined), and AH-R4 (5'-CAGTCCCGGGCTCTCTTGCGCCTGC-3' (SEQ ID NO: 23): XmaI site underlined). The thus-obtained DNA fragment was digested with restriction enzymes PstI and Xmal, and the fragment was inserted into each of pCAP-HA1/Vietnam, pCAP-HA1/Vietnam/272, and pCAP-HA1/Vietnam/467 which had been treated with restriction enzymes PstI and Xmal. The thus-constructed plasmids were denominated pCAP-HA1/Anhui, pCAP-HA1/Anhui/272, and pCAP-HA1/Anhui/467, respectively.
Note that the GenBank accession number of the amino acid sequence of hemagglutinin of influenza virus A/H5N1/Anhui is ABD28180.

### (5) Construction of transfer vector plasmid in which a gene of interest is not fused with a gp64 gene

### Construction of pCAP-bGal

PCR was performed by use of pCMV-SPORT-βGal (product of Invitrogen) as a template, forward primer bGal-F *PstI* F189-105 (ACTGCTGCAGATGTCGTTTACTTTGACCAACAAG) (SEQ ID NO: 24), and reverse primer bGal_Rev (*Dra*3) ACTGCACTTAGTGTTTTTGACACCAGACCAACTGG) (SEQ ID NO: 25). The thus-obtained DNA fragment was cloned between the PstI and DraIII sites of pCAP-HA1/Anhui. The thus-constructed plasmid was denominated pCAP-bGal.

### Construction of pCAP-Rluc

PCR was performed by use of pRL-SV40 (product of Promega) as a template, forward primer Rluc F PstI F189-134 (ACTGCTGCAGATGACTTCGAAAGTTTATGATCC) (SEQ ID NO: 26), and reverse primer Rluc R *Dra*III F189-134 (ACTGCACTTAGTGTTATTGTTCATTTTTGAGAACT) (SEQ ID NO: 27). The thus-obtained DNA fragment was cloned between the *Pst*I and *Dra*III sites of pCAP-HA1/Anhui. The thus-constructed plasmid was denominated pCAP-Rluc.

### Construction of pCAP-Luc

PCR was performed by use of pLuc-MCS (product of Stratagene) as a template, forward primer Rluc F PstI F189-105 (ACTGCTGCAGATGGGAGCTCGAATTCCAGCTTG) (SEQ ID NO: 28), and reverse primer Rluc-Rev2 F189-127 (ACTGCACTTAGTGTTACAATTTGGACTTTCCGCCC) (SEQ ID NO: 29). The thus-obtained DNA fragment was cloned between *Pst*I and DraIII sites of pCAP-HA1/Anhui. The thus-constructed plasmid was denominated pCAP-Luc.

### Construction of pCAP-AFP

PCR was performed by use of pQBI25-fA1 (product of MB Biomedical) as a template, forward primer AFP-F F189-77 (ACGCCTGCAGGCTAGCAAAGGAGAAGAACTCTTCA) (SEQ ID NO: 30), and reverse primer AFP-Rev2 F189-127 (ACTGCACTTAGTGTCAATCGATGTTGTACAGTTCA) (SEQ ID NO: 31). The thus-obtained DNA fragment was cloned between the PstI and DraIII sites of pCAP-HA1/Anhui. The thus-constructed plasmid was denominated pCAP-AFP.

### Construction of pCAP-Neo

PCR was performed by use of pcDNA3.1(+) (product of Invitrogen) as a template, forward primer Neo-F SbfI F189-105 (ACTGCCTGCAGGTGATTGAACAAGATGGATTGCAC) (SEQ ID NO: 32), and reverse primer Neo-Rev2 F189-127 (ACTGCACTTAGTGTCAGAAGAACTCGTCAAGAAGG) (SEQ ID NO: 33). The thus-obtained DNA fragment was cloned between PstI and DraIII sites of pCAP-HA1/Anhui. The thus-constructed plasmid was denominated pCAP-Neo.

### (6) Production of transfer vector plasmid pCAP-HA1/Vietnam/OG (6.1) Production of transfer vector plasmid pCAP-OG

pCAP-HA1/Vietnam was digested with restriction enzymes SpeI and DraIII. Into the fragment, a synthesized artificial gp64 gene sequence (SEQ ID NO: 34) digested with restriction enzymes SpeI and DraIII was inserted, to thereby construct a plasmid. The plasmid was denominated pCAP-OG.

### (6.2) Production of transfer vector plasmid pCAP-HA1/Vietnam/OG

pCAP-HA1/Vietnam was digested with restriction enzymes PstI and XmaI, to thereby prepare a fragment containing an HA1 gene. The fragment was inserted into pCAP-OG which had been digested with restriction enzymes PstI and XmaI. The thus-constructed plasmid was denominated pCAP-HA1/Vietnam/OG.

### (7) Production of transfer vector plasmids pCAP-CO/full/OG, pCAP-CO/76/OG, pCAP-CO/205/OG, and pCAP-CO/76/467

From an amino acid sequence of CSP of *Plasmodium falciparum* 3D7, an artificial gene sequence (SEQ ID NO: 35, PfCSP(3D7)_opt) was synthesized. PCR was performed by use of the artificial sequence as a template, and PfCSP_opt-f (5' - GACTCTGCAGATGATGCGAAAATTGGCCATACTG-3': PstI site underlined, SEQ ID NO: 36) and PfCSP_opt-r(397) (5' - CGATCCCGGGCATTGAGGAACAGAAAGGAAAGAACCATG-3': XmaI site underlined, SEQ ID NO: 37); PfCSP_opt-f(76) (5'-GACTCTGCAGGACGACGGAAATAATGAGGACAACG-3': PstI site underlined, SEQ ID NO: 38) and PfCSP_opt-r(373) (5' - CGATCCCGGGCCTTCTCCATCTTACAAATTTTCTTTTCAATATCATTAGC-3': XmaI site underlined, SEQ ID NO: 39); or PfCSP_opt-f(205) (5' - GACTCTGCAGAATGCAAACCCAAATGCCAATCCAAACGC-3': PstI site underlined, SEQ ID NO: 40) and PfCSP_opt-r(373) (5'-CGATCCCGGGCCTTCTCCATCTTACAAATTTTCTTTTCAATATCATTAGC-3': XmaI site underlined, SEQ ID NO: 39). Each of the obtained DNA fragments was digested with *Pst*I and *Xma*I, and the fragment was inserted into pCAP-HA1/Anhui or pCAP-HA1/Anhui/467 which had been treated with *Pst*I and *Xma*I. The thus-constructed plasmids were denominated pCAP-CO/full, pCAP-CO/76, pCAP-CO/76/467, and pCAP-CO/205, respectively.
Note that the GenBank accession number of the amino acid sequence of CSP of *Plasmodium falciparum* 3D7 is NC_000521.

### Example 2: Recombinant baculovirus of the present invention and production method therefor

By means of a recombinant baculovirus production kit (BacVector-2000 Transfection Kit, Novagen), recombinant baculoviruses were produced through co-transfection of Sf9 cells with BacVector-2000 DNA and each of the transfer vectors constructed in Example 1: pBACsurf-HA1/PR8, pCAP-HA1/PR8, pCAP-HA1/Vietnam, pCAP-HA1/Vietnam/51, pCAP-HA1/Vietnam/101, pCAP-HA1/Vietnam/154, pCAP-HA1/Vietnam/201, pCAP-HA1/Vietnam/272, pCAP-HA1/Vietnam/467, pCAP-HA1/Anhui, pCAP-HA1/Anhui/272, pCAP-HA1/Anhui/467, pCAP-HA1/Vietnam/OG, pCAP-bGal, pCAP-Rluc, pCAP-Luc, pCAP-AFP, and pCAP-Neo.
The thus-produced recombinant baculoviruses were denominated AcNPV-Pol-HA1/PR8, AcNPV-CAP-HA1/PR8, AcNPV-CAP-HA1/Vietnam, AcNPV-CAP-HA1/Vietnam/51, AcNPV-CAP-HA1/Vietnam/101, AcNPV-CAP-HA1/Vietnam/154, AcNPV-CAP-HA1/Vietnam/201, AcNPV-CAP-HA1/Vietnam/272, AcNPV-CAP-HA1/Vietnam/467, AcNPV-CAP-HA1/Anhui, AcNPV-CAP-HA1/Anhui/272, AcNPV-CAP-HA1/Anhui/467, AcNPV-CAP-HA1/Vietnam/OG, AcNPV-CAP-bGal, AcNPV-CAP-Rluc, AcNPV-CAP-Luc, AcNPV-CAP-AFP, and AcNPV-CAP-Neo, respectively.
Sf9 cells were cultured so that the cell concentration was adjusted to 2 × 10⁷ cells/plate (150-mm cell culture plate, Sumilon, product of Akita Sumitomo Bekelite Co., Ltd.), and the cells were infected with each of the aforementioned recombinant baculoviruses at a multiplicity of infection of about 0.1. After culturing for 5 days, the culture liquid was recovered by centrifugation at 10,000×g and 4°C for 25 minutes, followed by centrifugation by means of a Beckman ultra-centrifuge (SW28 Swing Rotor) at 25,000 rpm and 4°C for 90 minutes, to thereby recover virus particles.

### Example 3: Subculture of recombinant baculovirus

In a manner similar to that of Example 2, transfection was performed with each of the recombinant baculoviruses produced in Example 2 by means of a BacVector-2000 Kit (product of Novagen). After culturing for 5 days, the supernatant was recovered, and plaque purification was performed twice.
After the second purification, Sf9 cells inoculated to a 48-well plate were infected with the isolated plaque. After culturing for 5 days, the supernatant was recovered. Sf9 cells inoculated to a 25-cm² flask were infected with the supernatant at a multiplicity of infection of about 0.1. After culturing for 7 days, the supernatant was recovered (the recovered supernatant was employed as P2 virus stock). Sf9 cells were further infected with the P2 virus stock at a multiplicity of infection of about 0.1, and the culture supernatant recovered after 7 days culture was employed as P3 virus stock. During transfection and infection in the 48-well plate, a BacVector Insect Cell Medium (product of Novagen) was employed; during plaque purification, a 1.3×Sf900II medium (product of Invitrogen) was employed; and a TNM-FH Insect cell medium (product of Becton Dickinson) was employed during infection in the 25-cm² flask.

### Example 4: Recovery of genomic DNA from recombinant baculovirus, and PCR analysis of virus DNA

To each virus solution (50 µL), a DNA extraction buffer (50 mM Tris-HCl (pH: 8.0), 20 mM EDTA, 0.1 M NaCl, and 1% SDS) (400 µL), and 20 mg/mL proteinase K (8 µL) were added, and the mixture was incubated at 50°C for 3 hours. The solution was cooled to room temperature and then treated with phenol-chloroform (TE-saturated phenol : chloroform = 1 : 1, Wako Pure Chemical Industries, Ltd.) (458 µL). The genomic DNA of the virus was recovered from the supernatant through ethanol precipitation.

### (1) PCR analysis of virus DNA

PCR was performed by use of the recovered genomic DNA (1 ng) of the virus as a template.
PCR cycle was 94°C for two minutes, 30 cycles of 98°C for 10 seconds, 60°C for 30 seconds, and 68°C for 5 minutes, followed by 4°C. An aliquot (10 µL) of the PCR reaction mixture was subjected to agarose gel electrophoresis, and DNA was stained with ethidium bromide.
The combinations of primers employed in the PCR were as follows: forward primer Pol h-F (5'-CCATCTCGCAAATAAATAAGTA-3' (SEQ ID NO: 41)) and reverse primer Pol A-f (5'-AGAAGTCAGATGCTCAAG-3' (SEQ ID NO: 42)); forward primer CAG-F-FseI (5'-CTAGTTATTAATAGTAAT-3' (SEQ ID NO: 43)) and reverse primer GP64-R2 (5'-CATCGCCACGATTTGTTGCAC-3' (SEQ ID NO: 44)); and forward primer Pol h-F (5'-CCATCTCGCAAATAAATAAGTA-3' (SEQ ID NO: 41) and reverse primer GP64-R2 (5'-CATCGCCACGATTTGTTGCAC-3' (SEQ ID NO: 44)).

A PCR product was extracted from the agarose gel through irradiating the ethidium bromide-stained gel with UV light, cutting a DNA band of interest, and extracting DNA from the gel by means of a QIAquick Gel Extraction Kit (product of QIAGEN).
Sequencing of the PCR product was performed through extracting a PCR-amplified target DNA from the gel and employing the extract as a template for sequence analysis. Alternatively, the extract was cloned by means of a Zero Blunt TOPO PCR Cloning Kit (product of Invitrogen) and then employed as a template for sequence analysis. Cycle reaction was performed by use of DNA (5 to 300 ng) as a template and BigDye Terminator v3.1 (product of Applied Biosystems). The employed primers were Pol h-F (5'-CCATCTCGCAAATAAATAAGTA-3' (SEQ ID NO: 41)) and Pol A-f (5'-AGAAGTCAGATGCTCAAG-3' (SEQ ID NO: 42)).
The employed sequencer was Applied Biosystems 3130xl Genetic Analyzer (product of Applied Biosystems).

Subsequently, gene fragments for producing probes to detect restriction enzyme-treated virus DNA fragments in Southern blot analysis performed in the Examples hereinbelow were amplified through PCR. The target probes were as follows: an HA1/VN probe (which can detect a fusion gene of an antigen gene HA1 and a gp64 gene); a gp64 probe (which can detect a fusion gene of an antigen gene HA1 and a gp64 gene, a gene (mutant virus DNA) formed through removal of the antigen gene from the fusion gene, and an endogenous gp64 gene); and a promoter probe (which can detect a fusion gene of an antigen gene HA1 and a gp64 gene and a gene formed through removal of the antigen gene from the fusion gene). The promoter probe was produced, since the aforementioned gp64 probe cannot bind to a virus DNA fragment which includes a polynucleotide obtained through modification of a polynucleotide sequence of a naturally occurring gp64 gene, or a virus DNA fragment which includes a polynucleotide obtained through shortening the DNA sequence of the gp64 gene. The concentration of each amplified gene fragment was measured, and the fragment was used as a probe in an amount of 50 to 100 ng with respect to 10 mL of hybridization buffer.

### (2) Production of probes employed in Example 5 (I)

In order to produce a DNA fragment of the HA1/VN probe, PCR was performed by use of 10 µM forward primer VN-F2 (5'-GTGTCCAGCGCCTGCCCCTACCAGG-3' (SEQ ID NO: 45)), 10 µM reverse primer VN-R3 (5'-CCGTACTCCAGTTCTGACTTCATG-3' (SEQ ID NO: 46)), and a transfer plasmid pCAP-HA1/vietnam as a template DNA. In order to produce the gp64 probe, PCR was performed by use of 10 µM forward primer GP64-271-F (5'-ACCGAGTCAAGAAGCGGCC-3' (SEQ ID NO: 47)), 10 µM reverse primer pol A-f (5'-AGAAGTCAGATGCTCAAG-3' (SEQ ID NO: 42)), and a transfer plasmid pCAP-HA1/PR8 as a template DNA. Through the above PCR procedures, gene fragments for producing probes were obtained. The nucleotide sequences of the DNA fragments are shown in SEQ ID NOs.: 50 and 51.
The probes were produced from the gene fragments by means of a kit; an AlkPhos Direct Labeling and Detection System (GE Healthcare).

### (3) Production of probes employed in Example 8 (II)

The same HA1/VN probe and gp64 probe as employed in Example 5 were used. In order to produce the promoter probe, PCR was performed by use of 10 µM forward primer CAP-PromF F213-22 (5'-GCCTCTGCTAACCATGTTCATGC-3' (SEQ ID NO: 48)), 10 µM reverse primer CAP-PromR F213-22 (5'-CTTGCTTGTGTGTTCCTTATTGAAGCC-3' (SEQ ID NO: 49)), and a transfer plasmid pCAP-HA1/vietnam as a template DNA.
Through the above procedure, DNA fragments of the HA1/VN probe and the gp64 probe, and a gene fragment for producing the promoter probe were obtained. The nucleotide sequences of each DNA fragments are shown in SEQ ID NO: 50, 51, and 52.
The probes were produced from the gene fragments by means of a kit; an AlkPhos Direct Labeling and Detection System (product of GE Healthcare).

In a manner similar to that of Example 3, recombinant viruses were subcultured, and the amount of produced virus DNA was determined through PCR. Through Western blot analysis, a drop in expression amount of a target antigen was observed.
In order to confirm the cause for the drop in expression amount, PCR was performed by use of a primer set to amplify a region including a promoter sequence and an antigen sequence (CAG-F-FseI and GP64-R2), and virus genomic DNA prepared from each virus as a template (5 µL). After PCR, the PCR products were analyzed through agarose gel electrophoresis. As shown in Fig. 1, an unexpected band (lower) was observed at a size different from a deduced size (upper). This unexpected band was cut from the gel, and was subjected to sequence analysis, whereby a sequence containing no antigen gene in which a promoter sequence was ligated to a sequence of the gp64 gene was identified. This sequence suggests that the recombinant virus includes a mutant virus lacking an antigen gene.

As a positive control, plasmid pCAP-HA1/PR8 was employed (lanes 1 and 2). When the genomic DNA (100 ng) derived from Sf9 cells was employed, no such unexpected band was observed (lane 8). Thus, the obtained unexpected band was thought to be attributable to a recombinant baculovirus. Therefore, the recombinant virus, which also serves as an active ingredient of a medicament, was confirmed to be lowered in purity.
In the agarose gel electrophoresis, an aliquot (10 µL) of the reaction mixture, in which the template content was 5 µL/50 µL, was loaded on agarose gel.

Fig. 1 shows the results of PCR employing an annealing temperature of 60°C: lane 1, PCR product from 5 pg of plasmid pCAP-HA1/PR8; lane 2, PCR product from 0.5 pg of plasmid pCAP-HA1/PR8; lane 3, molecular weight marker (1kb Plus DNA ladder (product of Invitrogen)); lane 4, PCR product from virus DNA prepared from P2 virus stock; and each of lanes 5 to 7, PCR product from DNA of virus prepared through subculturing virus employed in lane 4 once more (P3 virus stock).

### Example 5: Detection of mutant virus through Southern blot analysis

A virus from the P3 virus stock of recombinant baculovirus AcNPV-CAP-HA1/Vietnam obtained through subculturing in Example 3 was subcultured once more. From the thus-obtained virus, virus DNA (0.2 µg) was recovered and digested with restriction enzymes *Swa*I (NEB) and *Xba*I (NEB). The whole digestion product was subjected to agarose gel electrophoresis.

The gel was treated with 0.25 M HCl solution, denaturation buffer (0.5 M NaOH, 1.5 M NaCl), and neutralization buffer (1 M Tris-HCl (pH: 7.5), 1.5 M NaCl). The DNA was transferred to a membrane by means of a Turbo Blotter (product of Whatman) and fixed through UV cross-linking (1200x100 uj/cm²).

Subsequently, the membrane was immersed in a hybridization buffer, to thereby perform prehybridization (hybridization buffer 10 mL/6×11 cm membrane) at 65°C for 30 minutes.
The hybridization buffer employed was a hybridization buffer included in a kit; AlkPhos Direct Labeling and Detection System (product of GE healthcare), and used according to an instruction attached to the kit.

The HA1/VN probe or gp64 probe produced in Example 4 was added to the hybridization liquid, and hybridization was performed at 65°C for 4 hours or longer.
After completion of hybridization, the membrane was washed, and bands were detected by use of a CDP-star detection reagent (product of GE Healthcare).
After detection of signals, re-probing was performed. Another hybridization was performed by use of a 1kb plus DNA ladder probe, which had been produced by labeling a 1kb plus DNA ladder (product of Invitrogen) (20 to 100 ng) by means of a kit; AlkPhos Direct Labeling and Detection System (product of GE Healthcare), to thereby determine the sizes of the bands. The results are shown in Figs. 2(A) and 2(B).

In Fig. 2, (A) shows the results of the case in which the HA1/VN probe was used, and (B) shows the results of the case in which the gp64 probe was used: lane 1, clone 1 of AcNPV-CAP-HA1/Vietnam; lane 2, another clone (clone 2) of AcNPV-CAP-HA1/Vietnam; lane 3, molecular weight marker (1kb plus DNA ladder (product of Invitrogen)); lane 4, clone 1 of AcNPV-CAP-HA1/Vietnam; lane 5, clone 2 of AcNPV-CAP-HA1/Vietnam; and lane 6, molecular weight marker (1kb plus DNA ladder (product of Invitrogen)).
As shown in Fig. 2(A), the HA gene was detected at the expected size. In Fig. 2(B), a band attributed to a fusion gene of the HA1 gene and the gp64 gene, and a band attributed to the endogenous gp64 gene (both bands being attributed to non-mutant virus) were observed. In Fig. 2(B), a band attributed to the gp64 gene (lacking HA1 gene) was also observed (the band being attributed to mutant virus). Thus, the presence of a mutant virus in the virus solution was confirmed.

### Example 6: Southern analysis of modified products of gp64 gene for preventing generation of mutant virus

Since the presence of the aforementioned mutant virus has been confirmed, the transfer vector produced in Example 1 (i.e., a plasmid vector having a polynucleotide sequence having a reduced identity of about 77% with a polynucleotide sequence encoding the amino acid residues constituting the naturally occurring GP64 protein so as not to alter the amino acid residues constituting the naturally occurring GP64 protein) was employed as a modified product of gp64 for preventing generation of mutant virus. By use of the plasmid vector, a recombinant baculovirus (AcNPV-CAP-HA1/Vietnam/OG) was produced. For the purpose of further refined analysis, some modified products of GP64 having a partially shortened GP64 nucleotide sequence were subjected to PCR in a similar manner.
Specifically, virus DNAs were recovered from P3 virus stock obtained through subculturing in Example 3, including AcNPV-CAP-HA1/Vietnam/OG, AcNPV-CAP-HA1/Vietnam, AcNPV-CAP-HA1/Vietnam/51, AcNPV-CAP-HA1/Vietnam/101, AcNPV-CAP-HA1/Vietnam/154, AcNPV-CAP-HA1/Vietnam/201, AcNPV-CAP-HA1/Vietnam/272, and AcNPV-CAP-HA1/Vietnam/467, in a manner as employed in Example 4. Each virus DNA was subjected to PCR analysis employing relevant primers. Fig. 3 shows the results.

In Fig. 3, lane 1 shows a molecular weight marker (1kb plus DNA ladder (product of Invitrogen)); lane 2 shows a band attributed to AcNPV-CAP-HA1/Vietnam/OG virus DNA; lane 3 shows bands attributed to AcNPV-CAP-HA1/Vietnam virus DNA; lane 4 shows bands attributed to AcNPV-CAP-HA1/Vietnam/51 virus DNA; lane 5 shows bands attributed to AcNPV-CAP-HA1/Vietnam/101 virus DNA; lane 6 shows bands attributed to AcNPV-CAP-HA1/Vietnam/154 virus DNA; lane 7 shows bands attributed to AcNPV-CAP-HA1/Vietnam/201 virus DNA; lane 8 shows bands attributed to AcNPV-CAP-HA1/Vietnam/272 virus DNA; and lane 9 shows a band attributed to AcNPV-CAP-HA1/Vietnam/467 virus DNA.

As is clear from Fig. 3, mutant virus was virtually or completely undetected in the cases of AcNPV-CAP-HA1/Vietnam/OG (i.e., a modified product obtained by reducing to about 77% the identity of the polynucleotide sequence of the gp64 gene with the polynucleotide sequence encoding the amino acid residues constituting naturally occurring GP64 protein so as not to alter the amino acid residues constituting the naturally occurring GP64 protein) and AcNPV-CAP-HA1/Vietnam/272 and AcNPV-CAP-HA1/Vietnam/467 (i.e., modified products obtained by partially shortening gp64 genes, in which the polynucleotide sequences were modified so that a portion of the amino acid residues constituting GP64 protein was deleted (49% and 87% amino acid sequences deleted, respectively)).

### Example 7: Southern analysis of modified products of gp64 gene for preventing generation of mutant virus

As clearly described above, generation of mutant viruses can be prevented or suppressed by constructing a transfer vector for a modified product by modifying the polynucleotide sequence of the gp64 gene so as not to alter the amino acid residues constituting the naturally occurring GP64 protein; or by constructing a transfer vector for a modified product by partially shortening the gp64 gene, in which the polynucleotide sequence was modified so that a portion of the amino acid residues constituting GP64 protein was deleted.
Then, generation of mutant viruses was studied when a transfer vector for a modified product (i.e., partially shortened gp64 gene), in which the immunogenic protein was changed to other influenza antigens, was employed.
Specifically, virus DNAs were recovered from P3 virus stock obtained through subculturing in Example 3, including AcNPV-CAP-HA1/Vietnam, AcNPV-CAP-HA1/Vietnam/272, AcNPV-CAP-HA1/Vietnam/467, AcNPV-CAP-HA1/Anhui, AcNPV-CAP-HA1/Anhui/272, and AcNPV-CAP-HA1/Anhui/467, in a manner as employed in Example 4. Each virus DNA was subjected to PCR analysis employing relevant primers. Fig. 4 shows the results.

In Fig. 4, lane 1 shows a molecular weight marker (1kb plus DNA ladder (product of Invitrogen)); lane 2 shows bands attributed to AcNPV-CAP-HA1/Vietnam virus DNA; lane 3 shows bands attributed to AcNPV-CAP-HA1/Vietnam/272 virus DNA; lane 4 shows bands attributed to AcNPV-CAP-HA1/Vietnam/467 virus DNA; lane 5 shows bands attributed to AcNPV-CAP-HA1/Anhui virus DNA; lane 6 shows bands attributed to AcNPV-CAP-HA1/Anhui/272 virus DNA; lane 7 shows bands attributed to AcNPV-CAP-HA1/Anhui/467 virus DNA; lane 8 shows bands attributed to AcNPV-CAP-HA1/Vietnam (mutant virus previously confirmed, positive control); each of lanes 9 to 13 shows a band for DNA obtained by PCR employing, as a template the genomic DNA of baculovirus AcNPV-CAP-bGal, AcNPV-CAP-R-Luc, AcNPV-CAP-Luc, AcNPV-CAP-AFP, or AcNPV-CAP-Neo, respectively, produced by use of a transfer vector in which the gp64 gene was not ligated to each target gene (pCAP-bGal, pCAP-Rluc, pCAP-Luc, pCAP-AFP, or pCAP-Neo); and lane 14 shows a molecular weight marker (1kb plus DNA ladder (product of Invitrogen)).

As is clear from Fig. 4, AcNPV-CAP-HA1/Vietnam/272, AcNPV-CAP-HA1/Vietnam/467, AcNPV-CAP-HA1/Anhui/272, and AcNPV-CAP-HA1/Anhui/467, in which the N-terminal amino acid sequence length of GP64 protein was shortened by 51% or 13% (gene level), exhibited a clear decrease in bands derived from mutant viruses (lanes 3, 4, 6 and 7), as compared with the constructs in which the N-terminal amino acid sequence length of GP64 protein was not shortened. In addition, when five clones (lanes 9 to 13) having a gene encoding a foreign immunogenic protein with which the gp64 gene was not fused were tested, no such mutant virus was detected. Furthermore, when the immunogenic protein was changed to other influenza antigens, no mutant virus was detected.

### Example 8: Detection of amounts of recombinant virus and mutant virus through Southern blot analysis

Virus DNAs were recovered from recombinant baculoviruses obtained through subculturing P3 virus stock obtained through subculturing in Example 3, including AcNPV-CAP-HA1/Vietnam, AcNPV-CAP-HA1/Vietnam/272, AcNPV-CAP-HA1/Vietnam/467, AcNPV-CAP-HA1/Vietnam/OG, and wild-type baculovirus (AcNPV). Each of the virus DNA was digested with restriction enzymes SwaI (NEB) and *Xba*I (NEB) and subjected to agarose gel electrophoresis.

Specifically, virus DNA (2 µg) was digested with a restriction enzyme *Swa*I (NEB) *and Xba*I (NEB), follwed by ethanol precipitation. The thus-obtained pellets were dissloved in a TE buffer, and the digestion product was separated by 0.8% agarose gel.

Subsequently, the gel was treated with 0.25 M HCl solution, denaturation buffer (0.5 M NaOH, 1.5 M NaCl), and neutralization buffer (1 M Tris-HCl (pH: 7.5), 1.5 M NaCl). The DNA contained in the gel was transferred to a membrane by means of a Turbo Blotter (product of Whatman) and fixed through UV cross-linking (1200x100 uj/cm²).
Subsequently, the membrane was immersed in a hybridization buffer, to thereby perform prehybridization (hybridization buffer 10 mL/6×11 cm membrane) at 65°C for 30 minutes. The hybridization buffer employed was a hybridization buffer included in a kit; AlkPhos Direct Labeling and Detection System (product of GE healthcare), and used according to an instruction attached to the kit.
The promoter probe produced in Example 4 was added to the hybridization liquid, and hybridization was performed at 65°C for 4 hours or longer.
After completion of hybridization, bands were detected by use of a CDP-star detection reagent (product of GE Healthcare).
After detection of signals, re-probing was performed. Another hybridization was performed by use of a 1kb plus DNA ladder probe, to thereby determine the sizes of the bands. The results are shown in Fig. 5 and Table 2.

In Fig. 5, lane 1 shows a band attributed to 1 × 10⁸ copies of pCAP-HA1/Vietnam plasmid (control) (0.84 ng); lane 2 shows a band attributed to 3 × 10⁷ copies of the control plasmid (0.252 ng); lane 3 shows a band attributed to 1 × 10⁷ copies of the control plasmid (0.084 ng); lane 4 shows a band attributed to a wild-type baculovirus DNA; lane 5 shows bands attributed to AcNPV-CAP-HA1/Vietnam/OG virus DNA; lane 6 shows bands attributed to AcNPV-CAP-HA1/Vietnam virus DNA; lane 7 shows bands attributed to AcNPV-CAP-HA1/Vietnam/272 virus DNA; and lane 8 shows bands attributed to AcNPV-CAP-HA1/Vietnam/467 virus DNA.

As is clear from Fig. 5, no mutant virus was detected in the cases of the recombinant baculovirus (AcNPV-CAP-HA1/Vietnam/OG), in which the polynucleotide sequence encoding GP64 protein was modified so as not to alter the amino acid residues constituting the naturally occurring GP64 protein; and a modified product (AcNPV-CAP-HA1/Vietnam/467), in which an N-terminal portion of the amino acid sequence constituting GP64 protein was considerably shortened, the two products falling within the scope of the present invention. However, bands attributed to mutant viruses were detected in the cases of AcNPV-CAP-HA1/Vietnam and AcNPV-CAP-HA1/Vietnam/272.
Then, the amount of expressed mutant virus was quantitatively determined based on the density ratio of the bands for non-mutant virus, mutant virus, and control plasmid. Table 2 shows the results.

**[Table 2]**

| | Mutant ratio (%) |
|---|---|
| AcNPV-CAP-HA1/Vietnam/OG | ND |
| AcNPV-CAP-HA1/Vietnam | 9.1 |
| AcNPV-CAP-HA1/Vietnam/272 | 0.8 |
| AcNPV-CAP-HA1/Vietnam/467 | ND |

| | |
|---|---|
| ND: No detection | |

As is clear from Table 2, the mutation was observed in about 9% of viruses in the case of AcNPV-CAP-HA1/Vietnam, in which the identity of the polynucleotide sequence of the gp64 gene had not been reduced, and in 0.8% of viruses in the case of AcNPV-CAP-HA1/Vietnam/272, in which only an N-terminal amino acid sequence of GP64 protein had been shortened. In addition, in the cases of AcNPV-CAP-HA1/Vietnam/OG and AcNPV-CAP-HA1/Vietnam/467, in which an N-terminal amino acid sequence of GP64 protein had been considerably shortened, the amount of mutant virus was under the detection limit (marked with ND (no detection) in Table 2).

In other words, since mutation of AcNPV-CAP-HA1/Vietnam/OG and AcNPV-CAP-HA1/Vietnam/467 were suppressed, the purity of a pharmaceutical substance containing as an active ingredient a recombinant baculovirus expressing a fusion product of an immunogenic antigen gene and the gp64 gene can be maintained. A drop in amount of expression of an immunogenic antigen, which is a member of the fusion product, caused by decrease in purity results in a reduced sensitization level of an administered antigen. Thus, the therapeutic effect by the antigen is problematically insufficient. However, through application of the production method according to the present invention, the aforementioned problem can be solved.

### Example 9: Western blot analysis of Sf9 cells infected with baculovirus

Variation in amount of generated mutant virus was investigated in the case where the transfer vector has a single promoter and in the case where the transfer vector has a dual promoter.
Recombinant baculovirus AcNPV-Pol-HA1/PR8 having a single promoter and recombinant baculovirus AcNPV-CAP-HA1/PR8 having a dual promoter, which were produced in Example 2, were used to produce plaques. In each case, eight plaques were collected. Separately, Sf9 cells were inoculated to a 48-well plate (product of Corning) and cultured. The cultured Sf9 cells were infected with a portion of the plaques. Five days after infection, the supernatant was removed, and Sf9 cells remaining on the plate was lyzed with 2xSDS sample buffer (5 mM Tris-HCl(pH: 7.0), 20% glycerol, 4% SDS, 0.004% BPB (Bromophenol Blue), and 10% 2-mercaptoethanol). The cell solution was transferred to an Eppendorf tube, followed by boiling for five minutes.

The solution was subjected to SDS-PAGE. Proteins were transferred to an Immubilon-P membrane (product of Millipore), and Western blot analysis was performed by use of an anti-GP64 antibody (product of e-Bioscience, 5,000-fold diluted) and a secondary antibody, Goat Anti-Mouse IgG (H+L) HRP Conjugate (product of BIORAD, 5,000-fold diluted), to thereby detect a fusion protein of GP64 and an antigen, and an endogenous GP64 protein.
There were used an SDS-PAGE electrophoresis buffer containing 25 mM Tris, 192 mM glycine, and 1% SDS, a blotting buffer containing 48 mM Tris (pH: 9.2), 39 mM glycine, 0.038% SDS, and 20% methanol, and, as a detection reagent, high-sensitivity chemiluminescence detecting ECL plus (product of GE Healthcare). Fig. 6 shows the results of Western analysis.

In the left photograph of Fig. 6, lanes 1 to 8 show clones 1 to 8 of recombinant virus AcNPV-CAP-HA1/PR8 produced from a transfer vector having a dual promoter; and lanes 9 to 16 show clones 1 to 8 of recombinant virus AcNPV-Pol-HA1/PR8 produced from a transfer vector having a polyhedrin promoter as a single promoter.

The right photographs (upper and lower) of Fig. 6 show the results of PCR analysis of virus DNAs prepared from the above-isolated clones. In the right upper and lower photographs of Fig. 6, lanes 17 and 27 show a molecular weight marker; lanes 18 to 24 show clones 2 to 8 of recombinant virus AcNPV-CAP-HA1/PR8 produced from a transfer vector having a dual promoter (not including clone 1, which was employed in another test); lanes 28 to 34 show clones 2 to 8 of recombinant virus AcNPV-Pol-HA1/PR8 produced from a transfer vector having a polyhedrin promoter as a single promoter (not including clone 1, which was employed in another test); lanes 25 and 35 show a positive control; and lanes 26 and 36 show a negative control.
As is clear from Fig. 6, both AcNPV-CAP-HA1/PR8 and AcNPV-Pol-HA1/PR8 generated mutant viruses, regardless of the type of promoter.

### Example 10: Analysis of characteristics of gp64 gene codon-modified baculovirus by use of a baculovirus to which human malaria (Plasmodium falciparum) CSP antigen was incorporated

Recombinant baculoviruses containing a malaria antigen (obtained through subculturing P3 virus stock); AcNPV-CO/full/OG, AcNPV-CO/76/OG, AcNPV-CO/205/OG, and AcNPV-CO/76/467, were purified and then denatured with 2xSDS sample buffer. The product was subjected to SDS-PAGE, transferred to membrane, and immunostained with an anti-GP64 antibody (product of e-Bioscience, 5,000-fold diluted) and a secondary antibody, sheep anti-mouse IgG (H+L) HRP Conjugate (product of BIORAD, 5,000-fold diluted), to thereby detect a fusion protein of GP64 and an antigen, and an endogenous GP64 protein. For attaining high sensitivity, immunostaining was performed with an anti-CSP antibody MRA-183 (MR4, 10,000-fold diluted) and a secondary antibody, sheep anti-mouse IgG (H+L) HRP Conjugate (product of BIORAD, 5,000-fold diluted), to thereby detect a fusion protein of GP64 and the antigen.

In Fig. 7, lanes 1 to 4 show the results of immunostaining by use of the anti-GP64 antibody; and lanes 5 to 8 show the results of immunostaining by use of the anti-CSP antibody. AcNPV-CAP-CO/full/OG was loaded on lanes 1 and 5, AcNPV-CAP-CO/76/OG was loaded on lanes 2 and 6, AcNPV-CAP-CO/205/OG was loaded on lanes 3 and 7, and AcNPV-CAP-CO/76/467 was loaded on lanes 4 and 8. As shown in Fig. 7, although the amounts of GP64-antigen fusion proteins relative to the amount of endogenous GP64 protein vary depending on the type of antigen, expression of GP64-antigen fusion protein was observed in all clones, even in the cases where codons of the gp64 gene were substituted.

In order to confirm that these recombinant viruses generated no mutant virus, the genomic DNA was prepared from each virus and subjected to PCR by use of Pol h-f primer and Pol A-f primer. Fig. 8 shows the results: lane 1, 1kb Plus DNA ladder; lane 2, PCR product from AcNPV-CAP-CO/full/OG; lane 3, PCR product from AcNPV-CAP-CO/76/OG; lane 4, PCR product from AcNPV-CAP-CO/205/OG; lane 5, PCR product from AcNPV-CAP-CO/76/467; lane 6, PCR product from AcNPV-CAP-HA1/Vietnam/OG as a negative control; and lane 7, PCR product from AcNPV-CAP-HA1/Vietnam as a positive control. Except the positive control, no mutant virus was detected.

## Claims

1. A method for producing a transfer vector which includes therein a fusion gene containing at least one gene encoding a virus-particle-forming protein and a gene encoding a foreign immunogenic protein, the method comprising modifying the polynucleotide sequence of the gene encoding the virus-particle-forming protein so as not to alter the amino acid residues constituting a corresponding naturally occurring virus-particle-forming protein and/or modifying the polynucleotide sequence of the gene so that a portion of the amino acid residues constituting the naturally occurring virus-particle-forming protein is deleted.

2. A method for producing a transfer vector which includes therein a fusion gene containing at least one gene encoding a virus-particle-forming protein and a gene encoding a foreign immunogenic protein, the method comprising modifying the polynucleotide sequence of the gene encoding the virus-particle-forming protein so as not to alter the amino acid residues constituting a corresponding naturally occurring virus-particle-forming protein.

3. A method according to claim 1, which comprises the following steps (a) to (c) and/or (d) and (e):
(a) a step of modifying the polynucleotide sequence of the gene encoding the virus-particle-forming protein so as not to alter the amino acid residues constituting a corresponding naturally occurring virus-particle-forming protein, and obtaining the nucleic acid sequence information of a polynucleotide including the gene encoding the virus-particle-forming protein;
(b) a step of synthesizing the polynucleotide on the basis of the nucleic acid sequence information obtained in step (a);
(c) a step of ligating or inserting, into the polynucleotide synthesized in step (b), a polynucleotide of the gene encoding the foreign immunogenic protein;
(d) a step of synthesizing a polynucleotide encoding a. partial polypeptide of the naturally occurring virus-particle-forming protein obtained through deletion of a portion of the amino acid residues constituting the naturally occurring virus-particle-forming protein, or a polynucleotide whose sequence has been modified so as not to alter the amino acid residues constituting the partial polypeptide; and
(e) a step of ligating or inserting, into the polynucleotide synthesized in step (d), a polynucleotide of the gene encoding the foreign immunogenic protein.

4. A method according to claim 1 or 2, wherein the fusion gene includes, upstream thereof, a polynucleotide encoding a signal sequence derived from the virus-particle-forming protein.

5. A method according to claim 1 or 2, wherein the gene encoding the virus-particle-forming protein is any of a baculovirus gp64 gene, a vesicular stomatitis virus glycoprotein gene, a human immunodeficiency virus type 1 glycoprotein gene, a human respiratory syncytial virus membrane glycoprotein gene, an influenza A virus hemagglutinin gene, an influenza B virus hemagglutinin gene, a herpes simplex virus glycoprotein gene, and a murine hepatitis virus S protein gene.

6. A method according to claim 1 or 2, wherein the gene encoding the virus-particle-forming protein is a baculovirus gp64 gene.

7. A method according to claim 1 or 2, wherein the gene encoding the foreign immunogenic protein is a gene for an antigen selected from among a malaria antigen, an influenza virus antigen, a *Mycobacterium tuberculosis* antigen, an SARS virus antigen, a West Nile fever virus antigen, a dengue fever virus antigen, an HIV antigen, an HCV antigen, a leishmania antigen, a trypanosome antigen, a leucocytozoon antigen, and a cancer antigen; or a gene for a fusion antigen of a cytokine and at least one gene selected from the group of these antigen genes.

8. A method according to claim 1 or 2, wherein, in the transfer vector, the fusion gene is ligated to the downstream of a dual promoter prepared through ligation between a promoter capable of functioning in vertebrate cells and a baculovirus promoter.

9. A method according to claim 8, wherein the promoter capable of functioning in vertebrate cells is selected from among a cytomegalovirus promoter, an SV40 promoter, a retrovirus promoter, a metallothionein promoter, a heat-shock protein promoter, a CAG promoter, an elongation factor 1α promoter, an actin promoter, a ubiquitin promoter, an albumin promoter, and an MHC class promoter; and the baculovirus promoter is selected from among a polyhedrin promoter, a p10 promoter, an IE1 promoter, a p35 promoter, a p39 promoter, and a gp64 promoter.

10. A method for producing a transfer vector containing a DNA region of a gene encoding a foreign immunogenic protein, the DNA region being inserted between virus DNA regions including at least one gene encoding a virus-particle-forming protein, the method comprising modifying a polynucleotide sequence of an N-terminal and/or C-terminal virus DNA region fused with the DNA region of the gene encoding the foreign immunogenic protein so that the polynucleotide sequence exhibits reduced identity to a naturally occurring polynucleotide sequence of the same virus DNA region; and/or modifying the polynucleotide sequence so that a portion of the amino acid residues constituting a corresponding naturally occurring virus-particle-forming protein is deleted.

11. A method for producing a recombinant baculovirus comprising employing a transfer vector which includes therein a fusion gene containing at least one gene encoding a virus-particle-forming protein and a gene encoding a foreign immunogenic protein, the method comprising co-transfecting, into an insect host cell, baculovirus DNA and the transfer vector produced by modifying the polynucleotide sequence of the gene encoding the virus-particle-forming protein so as not to alter the amino acid residues constituting a corresponding naturally occurring virus-particle-forming protein, and/or modifying the polynucleotide sequence of the gene so that a portion of the amino acid residues constituting the naturally occurring virus-particle-forming protein is deleted.

12. A transfer vector which includes therein a fusion gene containing at least one gene encoding a virus-particle-forming protein and a gene encoding a foreign immunogenic protein, wherein the polynucleotide sequence of the gene encoding the virus-particle-forming protein is modified so as not to alter the amino acid residues constituting a corresponding naturally occurring virus-particle-forming protein and/or the polynucleotide sequence of the gene is modified so that a portion of the amino acid residues constituting the naturally occurring virus-particle-forming protein is deleted.

13. A transfer vector which includes therein a fusion gene containing at least one gene encoding a virus-particle-forming protein and a gene encoding a foreign immunogenic protein, wherein the polynucleotide sequence of the gene encoding the virus-particle-forming protein is modified so as not to alter the amino acid residues constituting a corresponding naturally occurring virus-particle-forming protein.

14. A transfer vector according to claim 12, which is produced by performing the following steps (a) to (c) and/or (d) and (e):
(a) a step of modifying the polynucleotide sequence of the gene encoding the virus-particle-forming protein so as not to alter the amino acid residues constituting a corresponding naturally occurring virus-particle-forming protein, and obtaining the nucleic acid sequence information of a polynucleotide including the gene encoding the virus-particle-forming protein;
(b) a step of synthesizing the polynucleotide on the basis of the nucleic acid sequence information obtained in step (a);
(c) a step of ligating or inserting, into the polynucleotide synthesized in step (b), a polynucleotide of the gene encoding the foreign immunogenic protein;
(d) a step of synthesizing a polynucleotide encoding a partial polypeptide of the naturally occurring virus-particle-forming protein obtained through deletion of a portion of the amino acid residues constituting the naturally occurring virus-particle-forming protein, or a polynucleotide whose sequence has been modified so as not to alter the amino acid residues constituting the partial polypeptide; and
(e) a step of ligating or inserting, into the polynucleotide synthesized in step (d), a polynucleotide of the gene encoding the foreign immunogenic protein.

15. A transfer vector according to claim 12 or 13, wherein the fusion gene includes, upstream thereof, a polynucleotide encoding a signal sequence derived from the virus-particle-forming protein.

16. A transfer vector according to claim 12 or 13, wherein the gene encoding the virus-particle-forming protein is any of a baculovirus gp64 gene, a vesicular stomatitis virus glycoprotein gene, a human immunodeficiency virus type 1 glycoprotein gene, a human respiratory syncytial virus membrane glycoprotein gene, an influenza A virus hemagglutinin gene, an influenza B virus hemagglutinin gene, a herpes simplex virus glycoprotein gene, and a murine hepatitis virus S protein gene.

17. A transfer vector according to claim 12 or 13, wherein the gene encoding the virus-particle-forming protein is a baculovirus gp64 gene.

18. A transfer vector according to claim 12 or 13, wherein the gene encoding the foreign immunogenic protein is a gene for an antigen selected from among a malaria antigen, an influenza virus antigen, a *Mycobacterium tuberculosis* antigen, an SARS virus antigen, a West Nile fever virus antigen, a dengue fever virus antigen, an HIV antigen, an HCV antigen, a leishmania antigen, a trypanosome antigen, a leucocytozoon antigen, and a cancer antigen; or a gene for a fusion antigen of a cytokine and at least one gene selected from the group of these antigen genes.

19. A transfer vector according to claim 12 or 13, wherein the fusion gene is ligated to the downstream of a dual promoter prepared through ligation between a promoter capable of functioning in vertebrate cells and a baculovirus promoter.

20. A transfer vector according to claim 19, wherein the promoter capable of functioning in vertebrate cells is selected from among a cytomegalovirus promoter, an SV40 promoter, a retrovirus promoter, a metallothionein promoter, a heat-shock protein promoter, a CAG promoter, an elongation factor 1α promoter, an actin promoter, a ubiquitin promoter, an albumin promoter, and an MHC class promoter; and the baculovirus promoter is selected from among a polyhedrin promoter, a p10 promoter, an IE1 promoter, a p35 promoter, a p39 promoter, and a gp64 promoter.

21. A transfer vector containing a DNA region of a gene encoding a foreign immunogenic protein, the DNA region being inserted between virus DNA regions including at least one gene encoding a virus-particle-forming protein, wherein a polynucleotide sequence of an N-terminal and/or C-terminal virus DNA region fused with the DNA region of the gene encoding the foreign immunogenic protein is modified so that the polynucleotide sequence exhibits reduced identity to a naturally occurring polynucleotide sequence of the same virus DNA region; and/or the polynucleotide sequence is modified so that a portion of the amino acid residues constituting a corresponding naturally occurring virus-particle-forming protein is deleted.
